# EUROPÄISCHE PATENTSCHRIFT

(11) **EP 1 567 643 B1**
(45) Veröffentlichungstag und Bekanntmachung des Hinweises auf die Patenterteilung: **05.03.2008**
(21) Anmeldenummer: 03780122.2
(22) Anmeldetag: 04.12.2003
(51) Int. Cl.: C12N 15/10, C12N 15/62, C12Q 1/02, G01N 33/68

(54) **GEMISCH MINDESTENS ZWEIER FUSIONSPROTEINE SOWIE IHRE HERSTELLUNG UND VERWENDUNG**
MIXTURE OF AT LEAST TWO FUSION PROTEINS, THE PRODUCTION THEREOF AND THE USE OF THE SAME
MELANGE D'AU MOINS DEUX PROTEINES DE FUSION, SA PREPARATION ET SON UTILISATION

(30) Priorität: 04.12.2002 DE 10256669
(43) Veröffentlichungstag der Anmeldung: 31.08.2005
(73) Patentinhaber: Universitätsklinikum Charite Berlin, 10117 Berlin (DE)
(72) Erfinder: PASCHKE, Matthias, 10829 Berlin (DE)
(74) Vertreter: Zwicker, Jörk
(86) Internationale Anmeldenummer: PCT/EP2003/013709
(87) Internationale Veröffentlichungsnummer: WO 2004/050871

(56) Entgegenhaltungen:
- WO-A-00/71694
- WO-A-02/22667
- WO-A-92/18619
- WO-A-97/32017
- CRAMERI R ET AL: "DISPLAY OF BIOLOGICALLY ACTIVE PROTEINS ON THE SURFACE OF FILAMENTOUS PHAGES: A CDNA CLONING SYSTEM FOR SELECTION OF FUNCTIONAL GENE PRODUCTS LINKED TO THE GENETIC INFORMATION RESPONSIBLE FOR THEIR PRODUCTION" GENE, ELSEVIER BIOMEDICAL PRESS. AMSTERDAM, NL, Bd. 137, 1993, Seiten 69-75, XP000982171 ISSN: 0378-1119
- DELISA M P ET AL: "Genetic analysis of the twin arginine translocator secretion pathway in bacteria" JOURNAL OF BIOLOGICAL CHEMISTRY, AMERICAN SOCIETY OF BIOLOGICAL CHEMISTS, BALTIMORE, MD, US, Bd. 277, Nr. 33, 16. August 2002 (2002-08-16), Seiten 29825-29831, XP002971647 ISSN: 0021-9258 in der Anmeldung erwähnt
- PASCHKE MATTHIAS ET AL: "New series of vectors for phage display and prokaryotic expression of proteins" BIOTECHNIQUES, Bd. 30, Nr. 4, April 2001 (2001-04), Seiten 720-726, XP002276383 ISSN: 0736-6205 in der Anmeldung erwähnt
- DALBEY R E ET AL: "Protein translocation into and across the bacterial plasma membrane and the plant thylakoid membrane" TIBS TRENDS IN BIOCHEMICAL SCIENCES, ELSEVIER PUBLICATION, CAMBRIDGE, EN, Bd. 24, Nr. 1, Januar 1999 (1999-01), Seiten 17-22, XP004155514 ISSN: 0968-0004
- SETTLES A M ET AL: "Old and new pathways of protein export in chloroplasts and bacteria." TRENDS IN CELL BIOLOGY. ENGLAND DEC 1998, Bd. 8, Nr. 12, Dezember 1998 (1998-12), Seiten 494-501, XP002276384 ISSN: 0962-8924 in der Anmeldung erwähnt
- SAMUELSON J C ET AL: "YidC mediates membrane protein insertion in bacteria." NATURE. ENGLAND 10 AUG 2000, Bd. 406, Nr. 6796, 10. August 2000 (2000-08-10), Seiten 637-641, XP002276385 ISSN: 0028-0836 in der Anmeldung erwähnt

## Beschreibung

Die vorliegende Erfindung betrifft ein Proteingemisch, enthaltend mindestens ein erstes Fusionsprotein, enthaltend ein Protein oder Proteinfragment, eine Interaktionsdomäne und eine Proteintranslokationssequenz, die bewirkt, daß das Fusionsprotein bei Expression in ein Bakterium in einem im wesentlichen ungefalteten Zustand durch die zytoplasmatische Membran transloziert wird, und mindestens ein zweites Fusionsprotein, enthaltend ein Protein oder Proteinfragment, eine Interaktionsdomäne und eine Proteintranslokationssequenz, die bewirkt, daß das Fusionsprotein bei Expression in einem Bakterium in einem im wesentlichen gefalteten Zustand durch die zytoplasmatische Membran transloziert wird, wobei die Interaktionsdomäne des ersten Fusionsproteins an die des zweiten Fusionsproteins binden kann.

Die Phagendisplay-Technologie wird heutzutage in vielen Bereichen der Biotechnologie zum Auffinden von Proteinen mit gewünschten Bindungseigenschaften und enzymatischen Aktivitäten verwendet (Forrer, P. et al. (1999) Current Opinion in Struct. Biol. 9:514-520 und Gao, C. et al. (2002) Proc. Natl. Acad. Sci. U.S.A. 99:12612-12616). Gleichermaßen wird die Technologie verwendet, um beispielsweise die Bindungseigenschaften, die enzymatischen Eigenschaften und/oder die thermodynamische Stabilität von bereits bekannten oder durch die Phagendispplay-Technologie isolierten Proteine zu verbessern (Forrer, P. et al. (1999) siehe oben). Die Grundlage für die Phagendisplay-Technologie liegt in der Beobachtung, daß bestimmte sogenannte nicht-lytische Bakteriophagen Bakterien lediglich infizieren und die Phagenpartikel nicht etwa durch Lyse des Bakteriums freisetzen, sondern die einzelnen Bestandteile des Bakteriophagens durch das Zytoplasma ins Periplasma und letztendlich auf die Bakterienzelloberfläche transportieren, wo dann der komplette Phage assembliert wird, der sich anschließend von der Bakterienzelle löst. Die Fusion eines interessierenden Proteins mit einem Phagenhüllprotein führt daher zum Export dieses Proteins aus dem bakteriellen Zytoplasma und der Präsentation auf der Oberfläche des Bakteriums. Für die Präsentation geeignete Phagenhüllproteine sind beispielsweise die aus dem M13-Phagemid stammenden pIII, pVI, pVII, pVIII und pIX (Gao, C. et al. (2002) siehe oben).

Der N-Terminus des Phagenhüllproteins ist nach außen gerichtet, so daß das fusionierte Protein N-terminal vom Phagenhüllprotein angeordnet sein muß, damit es auf der Phagenoberfläche präsentiert wird. Dies führt zu keinem Problem, wenn einzelne bereits bekannte Proteine mit einem der genannten Phagenhüllproteinen fusioniert werden sollen, da für diese Proteine START- und STOP-Codons bekannt sind. Es führt jedoch dann zu Problemen, wenn eine sogenannte Phagen-Bibliothek hergestellt werden soll, bei der die Phagenhüllproteine mit einer cDNA-Bibliothek fusioniert werden sollen. Das Problem liegt darin, daß die in cDNA-Bibliotheken enthaltenden kodierenden Nukleinsäuren in der Regel translationelle STOP-Codons am 3'-Ende enthalten da durch die Poly(A⁺)-Selektion der mRNA und durch das sich anschließende Oligo-(dT)-priming die resultierenden cDNAs immer die translationellen STOP-Codons enthalten. So liegt bei Fusion einer Oligo-(dT)-geprimten cDNA 5' vom Phagenhüllprotein immer ein STOP-Codon zwischen der cDNA und dem Phagenhüllprotein, wodurch wiederum die Expression eines Fusionsproteins aus dem cDNA kodierten Protein und dem Phagenhüllprotein verhindert wird. Daher wurde von Crameri, R. und Suter, M. (1993) Gene 137:69-75 ein neuartiges Klonierungs- und Expressionssystem entwickelt, das darauf basiert, daß die Interaktionsdomänen der beiden Oncoproteine cJun und cFos, die über ein Proteinmotiv von gleichmäßig beabstandeten Leuzinresten dem sogenannten "Leuzin-Zipper" eine starke Wechselwirkung zwischen den beiden Proteinen ausbilden (Landschulz et al. (1988) Science 240:1759-64), genutzt werden, um das jeweils separat exprimierte Phagenhüllprotein und das cDNA-kodierte Protein zu einem Heterodimer zu verbinden. Dazu wurde von einem LacZ-Promotor gesteuert, ein Fusionsprotein exprimiert, das am N-Terminus aus cJun und am C-Terminus aus einem Phagenhüllprotein (_{P}III) bestand und ein zweites Fusionsprotein, das am N-Terminus aus cFos und am C-Terminus aus einer cDNA-Bank bestand, wobei auch dieses Protein durch einen zweiten LacZ-Promotor gesteuert wurde. Durch die Interaktion von cJun und cFos über die jeweiligen Leuzin-Zipper im Periplasma eines Bakteriums wurde dadurch die Präsentation von Protein bzw. Proteinfragmenten, die durch cDNAs kodiert sind auf filamentösen Phagen möglich.

Beim Einsatz der Phagendisplay-Technologie gibt es das weitere Problem, daß die Assemblierung der Phagen und somit auch der Einbau der Fusionsproteine in die Phagenpartikel ausschließlich im Periplasma stattfindet (Rüssel et al. (1997) Gene 192(1):23-32). Um die jeweiligen Fusionsproteine ins Periplasma der Bakterienzelle zu exportieren, muß demnach gegebenenfalls gentechnisch eine Sec-Signalsequenz an das Fusionsprotein angefügt werden. Diese Signalsequenz bewirkt, daß die Fusionsproteine in einem im wesentlichen ungefalteten Zustand ins Periplasma transportiert werden. Eine beträchtliche Anzahl von Proteinen kann jedoch nicht mittels des Sec-Transportwegs ins Periplasma gelangen, da sogenannte "Stop-Transfer"-Sequenzen oder eine zu schnelle Faltung des Proteins, die bereits ins Zytoplasma erfolgt, einen Transport verhindern. "Stop-Transfer"-Sequenzen bewirken durch eine lokale Häufung positiv-geladener Aminosäuren in der Proteinsequenz, daß das entsprechende Protein bei der Translokation über den Sec-Transportweg in der inneren Membran stecken bleibt. Proteine, die durch ihre schnelle und/oder stabile Faltung nicht mehr in der entfalteten Form von den Proteinen des Sec-Transporiweges - insbesondere von SecB - gebunden werden können, werden nicht zum Sec-Tranlokase-Komplex transportiert und verbleiben im Cytoplasma (Yamane et al. (1988) J. Bio. Chem. 263:19690-19696 und Berks, B. C. (1996) Mol. Microbiol. 22:393-404 und Bergs, B. C. et al. (2000) Mol. Microbiol. 35:260-274). Proteine, die auf reduzierende Bedingungen oder die für ihre Funktionsfähigkeit auf zytoplasmatische Co-Faktoren, wie beispielsweise FeS-Zentren oder Molybdopterin angewiesen sind, können ebenfalls nicht über den Sec-Transportweg in funktioneller Form ins Periplasma gelangen. Aufgrund der Inkompatibilität mit dem Sec-Transportweg können demnach viele Polypeptide nicht funktionell gefaltet mit dem Phagendisplay präsentiert und anschließend selektiert werden. Die Translokation der Fusionsproteine über den Sec-Transportweg ins Periplasma stellt daher einen wesentlichen Nachteil der im Stand der Technik bekannten Phagendisplay-Techniken dar.

Aus den unterschiedlichen Anforderung an das zelluläre Milieu bei Faltung bestimmter Proteine ergibt sich ein weiteres Problem bei der Expression von Fusionsproteinen insbesondere in Bakterien, wenn der eine Teil des Fusionsproteins nur im Periplasma eine korrekte Faltung annimmt, wie das z.B. bei Antikörperproteinen der Fall ist (Gao, C. et al. (2002) siehe oben) und der andere Teil des Fusionsproteins nur im Zytoplasma korrekt gefaltet werden kann, wie das z.B. beim grün-floreszierenden Protein (GFP) der Fall ist, das Sec-inkompatibel ist. So ist z.B. die Expression von Antikörper-GFP-Fusionsprotein, d.h. von Fluoreszenz-markierten Antikörpermolekülen, in Bakterien bisher nicht möglich. Die Beschränkung auf den Sec-Transportweg verhindert somit die Herstellung einer Vielzahl interessanter Proteinkonjugate insbesondere in Bakterien.

WO 92/ 18619 A beschreibt rekombinante filamentöse Phagen, die das Matrixprotein cpVIII an ihrer Oberfläche besitzen, des Weiteren ein Genom beinhalten, das für ein erstes und zweites Polypeptid kodiert, wie z.B. Antikörper oder Rezeptoren und die in die Oberfläche des Phagen integriert werden, sobald diese als Fusionsproteine mit einem Phagenhüllprotein, das es in der äußeren Membran verankert, exprimiert werden.

Crameri R. et al. (1993) Gene 137:69-75 beschreibt ein Phagendisplaysystem, in dem zwei chimäre Proteine, (i) ein heterologes Genprodukt fusioniert mit Fos und (ii) das Phagenprotein pIII fusioniert mit Jun, exprimiert werden. Die Fusionsproteine werden durch pelB-Signalsequenzen in den periplasmatischen Raum transportiert, so dass letztlich das mit Fos fusionierte heterologe Genprodukt mit dem Jun-dekorierten Phagenpartikel an der Oberfläche interagieren kann.

Die WO 97/32017 A beschreibt eine Methode zur Identifikation von Nukleinsäuren, die über zwei oder mehrere Polypeptidketten miteinander interagieren können. So werden zwei verschiedene Genbanken mit bzw. ohne pIII fusioniert und in ein Bakterium eingebracht, selektiert und exprimiert, so dass die aus der ersten Genbank resultierenden Produkte, die in der Oberfläche der Phagen verankert sind, die Möglichkeit haben, mit den Produkten, die aus der anderen Genbank stammen, zu interagieren.

Die WO 00/71694 A beschreibt ein Phagendisplaysystem heteromerer Proteine an der Oberfläche von filamentösen Phagen, die mit pVII bzw. pIX fusioniert wurden. Dabei werden die beiden Signalsequenzen pe1B und ompA in einem dicistronischen Vektor mit den monomeren Polypeptiden verbunden und so in das Periplasma transportiert.

Eine Aufgabe der vorliegenden Erfindung besteht daher darin die Einschränkung der im Stand der Technik bekannten Phagendisplay-Technologie auszuräumen und die Herstellung von Fusionsproteinen zu ermöglichen, die bei Herstellung durch die im Stand der Technik bekannten Verfahren nicht zu funktionellen Fusionsproteinen fuhren.

Die vorliegende Erfindung stellt daher in einem Aspekt ein Proteingemisch zur Verfügung, enthaltend: a) mindestens ein erstes Fusionsprotein, enthaltend: i) ein Protein oder Proteinfragment, ii) eine Interaktionsdomäne und iii) eine Proteintranslokationssequenz, die bewirkt, daß das Fusionsprotein bei Expression in einem Bakterium in einem im wesentlichen ungefalteten Zustand durch die zytoplasmatische Membran transloziert wird, und b) mindestens ein zweites Fusionsprotein, enthaltend: i) ein Protein oder Proteinfragment, ii) eine Interaktionsdomäne und iii) eine Proteintranslokationssequenz, die bewirkt, daß das Fusionsprotein bei Expression in einem Bakterium in einem im wesentlichen gefalteten Zustand durch die zytoplasmatische Membran transloziert wird, wobei die Interaktionsdomäne des ersten Fusionsproteins an die des zweiten Fusionsproteins binden kann.

Das Protein oder Proteinfragment des ersten Fusionsproteins umfaßt vorzugsweise Proteine, die im ungefalteten Zustand durch die zytoplasmatische Membran eines Bakteriums, vorzugsweise eines Gram-negativen Bakteriums transloziert werden können und die demnach nicht für ihre korrekte Faltung auf das reduzierende zytoplasmatische Milieu und/oder auf zytoplasmatische Co-Faktoren angewiesen sind und die auch im Periplasma eine im wesentlichen korrekte Faltung erreichen können. Beispiele solcher Proteine umfassen, sind aber nicht limitiert auf schwere Immunoglobulinketten, leichte Immunoglobulinketten, Fragmente dieser Ketten, sogenannte "Single-Chain-Antikörper" (Bird, R. E. (1988)Science 242:423-6), Diabodies (Holliger, P. (1993) Proc. Natl. Acad. Sci. U.S.A 90(14):6444-8, Rezeptoren, vorzugsweise extrazelluläre Domänen von Rezeptoren, wie beispielsweise, EGFR, PDGFR oder VEGFR, oder Rezeptorliganden, wie beispielsweise EGF, PDGF oder VEGF, Integrine, vorzugsweise deren extrazelluläre Domänen, Intimine und deren Domänen, wie beispielsweise EaeA, Kohlenhydrat-bindende Proteine und Domänen davon, wie MBP und CBD, Albuminbindende Proteine und Domänen oder Protein A und dessen Domänen.

Das Protein oder Proteinfragment des zweiten Fusionsproteins kann ein beliebiges Protein oder Proteinfragment sein, bevorzugt sind jedoch Proteinfragmente, die ihre Faltung und/oder ihre Funktion nur dann erhalten, wenn sie sich bereits im Zytoplasma eines Bakteriums falten und daher in einen im wesentlichen gefalteten Zustand durch die zytoplasmatische Membran in das Periplasma transloziert werden. Beispiele solcher Proteine sind autofluoreszierende Proteine, wie beispielsweise GFP oder Varianten davon mit veränderten Absorptionsmaxima, Enzyme, wie beispielsweise β-Lactamase, Cofaktor-abhängige Proteine, wie beispielsweise TMAO-Reduktase und Meerretdch-Peroxidase, Proteine, die von einer cDNA kodiert werden, welche aus einer cDNA-Bibliothek stammt, oder synthetische Proteine.

In einer bevorzugten Ausführungsform ist das Protein oder Proteinfragment des ersten Fusionsproteins und die Proteintranslokationssequenz ein Phagenhüllprotein oder ein periplasmatisches Markerenzym, wie PhoA, ein Intimin ein Protein der äußeren Bakterienmembran oder ein periplasmatisches Rezeptorprotein, insbesondere ein Kohlenhydrat-bindendes Protein. Bevorzugte Phagenhüllproteine, die in einem Proteingemisch der vorliegenden Erfindung enthalten sein können, sind ausgewählt aus den M13-Phagemid Hüllproteinen pIII, pVI, pVII, pVIII und pIX. Von diesen Phagenhüllproteinen sind jedoch nur pIII und pVIII mit einer bekannten Sec-abhängigen Proteintranslokationssequenz versehen, wobei die in den restlichen Phagenhüllproteinen enthaltenden Proteintranslokationssequenzen noch nicht identifiziert worden sind. Da diese Phagenhüllprotein dennoch in einem im wesentlichen ungefalteten Zustand in das Periplasma des Bakteriums transportiert werden, werden solche Proteine im Sinn der Erfindung auch ohne Identifikation der Proteintranslokationssequenz als Proteine angesehen, die aus einem Protein oder Proteinfragment und einer Proteintranslokationssequenzen bestehen.

Die Interaktionsdomänen, die in dem ersten und zweiten Fusionsprotein verwendet werden, Rihren zur Bindung des ersten Fusionsproteins an das zweite Fusionsprotein. Bevorzugt sind hierbei Interaktionsdomänen, die zu einer relativ festen Interaktion der beiden Proteine führen, wobei eine relativ feste Interaktion eine Interaktion ist, die auch im oxidativen Milieu des Periplasmas, auf der Bakterienzelloberfläche oder bei des Sezernierung des Heterodimers oder Heteromultimers auch außerhalb der Zelle bestehen bleibt. Geeignete Interaktionsdomänen des ersten und zweiten Fusionsproteins, die erfindungsgemäß in den Fusionsproteinen enthalten sein können, sind beispielsweise eine Leuzin-Zipper-Domäne und eine Leuzin-Zipper-Domäne, wie sie zuerst in den zwei Oncoproteinen cJun und cFos beschrieben wurden (Landschulz et al. (1988) siehe oben) Varianten davon aus anderen Hetero- oder Homodimeren sowie arfifizielle Leuzin-Zipper-Domänen oder eine Helix-Loop-Helix-Domäne und eine Helix-Loop-Helix-Domäne (Moor et al. (1989) Cell 56:777-783), ein Calmodulin und ein Calmodulin-bindendes Peptid (Montigiani, S. et al. (1996) JMB 258:6-13) oder jeweils ein Peptid eines Peptid-Dimers. Der Begriff Interaktionsdomänen umfaßt auch Domänen die eine Multimerisierung von mehr als zwei Fusionsproteinen erlauben.

Die Proteintranslokationssequenz des ersten Fusionsproteins bewirkt, daß das Fusionsprotein bei Expression in einem Bakterium, vorzugsweise in einem Gram-negativen Bakterium in einem im wesentlichen ungefalteten Zustand durch die zytoplasmatische Membran in das Periplasma transloziert wird. Der Fachmann ist ohne weiteres in der Lage, entsprechende Proteintranslokationssequenzen aufzufinden, wobei er sich des folgenden Experiments bedienen kann. Eine potentielle als Proteintranslokationssequenz geeignete Proteinsequenz, die zur Translokation eines damit fusionierten Proteins in einem im wesentlichen ungefalteten Zustand führt, wird mit einem ein GFP-myc-TAG enthaltenden Protein fusioniert. Wenn die potentielle Proteintranslokationssequenz nicht zur Proteintranslokation ins Periplasma führt, wird das GFP-Protein im Zytoplasma des Bakteriums gebildet, was sich über die zytoplasmatische Fluoreszenz nachweisen läßt, es gelangt dann jedoch nicht an die Oberfläche oder ins Medium, so daß der Myc-TAG durch einen anti-Myc-Antikörper bspw. den monoklonalen Antikörper 9E10 weder im Medium noch auf der Oberfläche nachweisbar ist. Führt die Sequenz jedoch zur Translokation des Fusionsproteins ins Periplasma und schließlich zur Präsentation auf der Oberfläche bzw. zur Sezernierung in die Umgebung des Bakteriums, so läßt sich das präsentierte bzw. sezernierte GFP-myc-TAG-Fusionsprotein durch einen Anti-myc-Antikörper im Medium und/oder auf der Oberfläche des Bakteriums nachweisen. Gleichzeitig sollte im Periplasma in diesem Fall keine Fluoreszenz beobachtet werden können, da bei Translokation des GFPs ins Periplasma in einem im wesentlichen ungefalteten Zustand sich das Protein dort nicht mehr korrekt faltet (sogenannte "Sec-Inkompatibilität"). Die Proteintranslokationssequenzen die bevorzugt im ersten Fusionsprotein verwendet werden sind solche, die in dem Sec-abhängigen Transportweg (Danese, P. N. und Silhavy, T. J. (1998) Annu. Rev. Genet. 32:59-94), die in dem SRP-abhängigen Transportweg (Meyer, D. I. et al. (1982) Nature 297:647-650) oder die in einem YidC-abhängigen Transportweg erkannt werden (Samuelson, J. C. et al. (2000) Nature 406:637-641). Allerdings kann es sich auch um eine Transportweg-unabhängige Sequenz handeln. Besonders geeignete Proteintranslokationssequenzen sind daher beispielsweise Signalsequenzen des PhoA, PelB, OmpA und pIII.

Als weiterer Bestandteil enthält das zweite Fusionsprotein eine Proteintranslokationssequenz, die bewirkt, daß das Fusionsprotein bei Expression in einem Bakterium, vorzugsweise in einem Gram-negativen Bakterium, in einem im wesentlichen gefalteten Zustand durch die zytoplasmatische Membran transloziert wird. Eine Proteintranslokationssequenz mit dieser Eigenschaft liegt dann vor, wenn ein Protein, beispielsweise GFP, das nur im Zytoplasma des Bakteriums seine funktionelle Konformation einnehmen kann, ohne Verlust der Autoflureszenz ins Periplasma transportiert wird. Diese Eigenschaft der erfindungsgemäßen Proteintranslokationssequenz läßt sich mit dem vorangehend im Hinblick auf die erste Proteintranslokationssequenz beschriebenen Experiment, untersuchen. Mit einem ähnlichen Experiment wurde bereits ein Konsensusmotiv für das Tat-spezifische Führungspeptid des Twin-Argenine-Translokation-(Tat)-Transportwegs von Bakterien und Pflanzenchloroplasten ermittelt. Der im Stand der Technik bekannte Tat-Transportweg erlaubt den Transport von bereits im Zytoplasma gefalteten Proteinen ins Periplasma und kann somit Proteine, die mit dem Sec-Transportweg inkomatibel sind, ins Periplasma transportieren. Genau wie der Transport über den Sec-Transportweg, wird auch der Tat-Transprotweg durch eine spezielle Gruppe von Führungssequenzen vermittelt (DeLisa, M. P. et al. (2002) J. Biol. Chem. 277:29825-29831). Ein weiterer im Stand der Technik bekannter Transportweg, der den Transport von Proteinen in im wesentlichen gefalteten Zustand erlaubt, ist der über Thylakoid-Membranen (Settles, A. M. und Martienssen, R. (1998) Transcell Biol. 8:494-501). Daher enthält das zweite Fusionsprotein in einer bevorzugten Ausführungsform der vorliegenden Erfindung eine Signalsequenz, die von einem Tat-abhängigen Transportweg oder von einem Thylakoid-Aphabhängigen Transportweg erkannt wird und dadurch zur Translokation des Fusionsproteins in im wesentlichen gefalteten Zustand führt. Ein Konsensusmotiv einer Proteintranslokationssequenz, das von einem Tat-abhängigen Transportweg erkannt wird, ist in DeLisa, M. P. et al. ((2002) siehe oben) beschrieben worden. Die Sequenz lautet: S/T/RRXFLK.

In einer bevorzugten Ausführungsform des erfindungsgemäßen Proteingemisches sind mindestens ein erstes und mindestens ein zweites Fusionsprotein aneinander kovalent oder nicht-kovalent gebunden. Zur Erreichung einer kovalenten Bindung der zwei getrennt exprimierten Fusionsproteine können beispielsweise in dem Protein zusätzlich nahe der Interaktionsdomäne Cysteinreste oder Homologe davon angeordnet werden, die im oxidativen Milieu des Periplasmas eine kovalente Bindung zwischen den beiden Fusionsproteinen herstellen. Eine kovalente Bindung kann jedoch beispielsweise auch durch die Inkorporation von Aminosäuren mit Foto-aktivierbaren Gruppen in die beiden Fusionsproteine und anschließende UV-Exposition der zunächst lediglich nicht-kovalent aneinander gebundenen Protein erreicht werden. Dem Fachmann sind weitere Verfahren bekannt, um zwei zunächst allein durch nicht-kovalente Bindung verbundene Proteine miteinander zu verbinden. Zu diesem dem Fachmann bekannten Verfahren der kovalenten Verbindung zweier nicht-kovalentgebundener Fusionsproteine zählt beispielsweise das Psoralen-Crosslinking.

Ein weiterer Gegenstand der vorliegenden Erfindung ist ein Nukleinsäuregemisch, das für ein erfindungsgemäßes Proteingemisch kodiert. Eine kodierende Nukleinsäure, im Sinne der Erfindung ist ein Nukleinsäuresequenz, die für ein erfindungsgemäßes Polypeptid oder einen Vorläufer davon kodiert. Bevorzugterweise handelt es sich bei dem Nukleinsäuregemisch um DNA oder RNA, vorzugsweise um eine DNA, wobei die DNA einzelsträngig oder doppelsträngig vorliegen kann. Die jeweils für das erste oder zweite Fusionsprotein kodierende Nukleinsäure enthält des weiteren Promotoren, die die Expression des jeweiligen Fusionsproteins in der Wirtszelle ermöglichen. Geeignete Promotoren für die Expression in beispielsweise *E*. *coli* sind der trp-Promotor, lacZ-Promotor, tet-Promotor, T7-Promotor oder ara-Promotor. Weitere Elemente, die in den jeweiligen, das Nukleinsäuregemisch ausmachenden Nukleinsäuren vorhanden sein können, sind Replikationsursprünge (Ori), selektive Markergene, die beispielsweise Ampizilin- oder Chloramphenikolresistenz vermitteln. Die Nukleinsäuren können neben dem für das jeweilige Fusionsprotein kodierenden Bereich, die üblicherweise in bakteriellen Expressionsvektoren verwendeten Elemente aufweisen. Dem Fachmann sind eine Vielzahl solcher Elemente sowie Vektoren bekannt, wie beispielsweise pGEM oder pUC.

In einer bevorzugten Ausführungsformen des Nukleinsäuregemisches der vorliegenden Erfindung, sind die zwei Nukleinsäuren, die für das erste und zweite Fusionsprotein kodieren, kovalent miteinander verbunden, vorzugsweise über Phosphordiester-Bindungen. Insbesondere sind die Nukleinsäuremoleküle, die für das erste und zweite Fusionsprotein kodieren und geeignete regulatorische Elemente enthalten, auf einem Plasmid enthalten, so daß die erfindungsgemäßen Proteingemische bereits durch Transfektion nur eines Plasmids bzw. wenn diese Nukleinsäure in einem Phagen enthalten ist, durch Infektion mit nur einem Phagen beispielsweise in einem Bakterium -hergestellt werden können. In einer bevorzugten Ausfilhrungsform werden beide Fusionsprotein unter der Kontrolle nur eines Promotors als bicistronische Kassette exprimiert.

Ein weiterer Gegenstand der vorliegenden Erfindung ist ein Vektor, enthaltend ein erfindungsgemäßes Proteingemisch und/oder enthaltend ein erfindungsgemäßes Nukleinsäuregemisch. Ein Vektor, im Sinne der vorliegenden Erfindung, ist ein Protein-Nukleinsäuregemisch, das in der Lage ist, die in ihm enthaltenen Proteingemische und/oder Nukleinsäuregemisch in eine Zelle einzuführen. Dabei ist es bevorzugt, daß dabei die von den Nukleinsäuregemischen kodierten Fusionsproteine in der Zelle zur Expression kommen und somit de *novo* synthetisierte Fusionsproteine aus der Zelle gewonnen, bzw. auf der Zelloberfläche präsentiert werden. Geeignete Vektoren sind beispielsweise nicht-lytische Phagen, wie M13-Phage, fd-Phage, F1-Phage und lytische Phagen, wie λ-Phage.

Ein weiterer Gegenstand der vorliegenden Erfindung, ist eine Zelle, enthaltend ein erfindungsgemäßes Proteingemisch, ein erfindungsgemäßes Nukleinsäuregemisch und/oder einen erfindungsgemäßen Vektor. Erfindungsgemäße Zellen können prokaryontische oder eukaryontische Zellen sein. In einer bevorzugten Ausführungsform der vorliegenden Erfindung, handelt es sich bei den erfindungsgemäßen Zellen um prokaryontische Zellen, insbesondere um Bakterien und noch bevorzugter um *E. coli* (TG1, XL-1, JM83, BL21) oder *B. subtilis.*

Ein weiterer Gegenstand der vorliegenden Erfindung ist eine Bibliothek, enthaltend mindestens zwei erfindungsgemäße Proteingemische, mindestens zwei erfindungsgemäße Vektoren und/oder mindestens zwei erfindungsgemäße Zellen, wobei die Proteine oder Proteinfragmente der jeweiligen ersten oder jeweiligen zweiten Fusionsproteine voneinander unterschiedlich sind. Eine solche Bibliothek kann entweder gezielt ausgewählte unterschiedliche bekannte Proteine oder Proteinfragmente enthalten oder die Interaktionsdomäne und die Proteintranslokationssequenz des ersten oder zweiten bevorzugt des zweiten Fusionsproteins können mit einer cDNA-Bibliothek fusioniert werden, wobei bei Expression dieser Nukleinsäuren eine Vielzahl unterschiedlicher erster oder zweiter Fusionsproteine entstehen, die jeweils unterschiedliche Proteine oder Proteinfragmente enthalten. Vorzugsweise wird dabei der cDNA-Anteil am C-Terminus des Fusionsproteins exprimiert, um dadurch das vorangehend geschilderte Problem bei N-terminaler Fusion einer cDNA zu umgehen. In einer bevorzugten Ausführungsform enthält die Bibliothek eine Vielzahl von erfindungsgemäßen Zellen, wobei jede Zelle ein anderes Proteingemisch herstellt, vorzugsweise auf ihrer Oberfläche präsentiert. Im Falle, daß das Protein oder Proteinfragment und die Interaktionsdomäne des ersten Proteins ein Phagenhüllprotein ist, erlaubt die erfindungsgemäße Bibliothek die Präsentation einer Vielzahl von Proteinen oder Proteinfragmenten, die im zweiten Fusionsprotein enthalten sind. Dabei ist die Präsentation nicht wie bei den im Stand der Technik bekannten Phagendisplay-Bibliotheken auf Proteine oder Proteinfragmente beschränkt, die sich im Periplasma der Zelle zu ihrer funktionellen Form falten, sondern umfaßt auch Proteine, die nur im Zytoplasma ihre funktionelle Faltung erlangen können.

Die erfindungsgemäßen Proteingemische, die Heterodimere oder Multimere bilden können, wobei die Bestandteile der Heterodimere oder Multimere in mindestens zwei unterschiedlichen zellulären Kompartments ihre dreidimensionale Struktur erhalten haben, lassen sich nunmehr in einer Reihe von Verfahren unter anderem auch im Phagendisplay einsetzen.

Ein weiterer Gegenstand der Erfindung ist daher ein Verfahren zum Auffinden von Substanzen, die an ein Proteingemisch, einen erfindungsgemäßen Vektor oder an eine erfindungsgemäße Zelle binden, enthaltend die Schritte:
a) In-Kontaktbringen mindestens einer potentiell-bindenden Substanz mit einem erfindungsgemäßen Proteingemisch, einem erfindungsgemäßen Vektor oder einer erfindungsgemäßen Zelle und
b) Messen der Bindung der Substanz an das Proteingemisch, den Vektor und/oder die Zelle.

Das Verfahren dient somit vorzugsweise dem Auffinden einer Substanz oder von Substanzen, die an ein bereits bekanntes Proteintarget binden, beispielsweise um Inhibitoren, Aktivatoren, Kompetitoren oder Modulatoren des bekannten Proteintargets aufzufinden. Die potentiell bindenden Substanzen deren Bindung an ein erfindungsgemäßes Proteingemisch, einen erfindungsgemäßen Vektor und/oder eine erfindungsgemäße Zelle gemessen werden soll, kann jede beliebige chemische Substanz oder jedes beliebige Substanzgemisch sein. Beispielsweise kann es sich hierbei um Substanzen einer Peptid-Bibliothek handeln, um Substanzen aus einer kombinatorischen chemischen Bibliothek, um Zellextrakte, insbesondere Pflanzenzellextrakte, und um Proteine oder Proteinfragmente.

Unter In-Kontaktbringen der potentiell bindenden Substanz(en) mit einem erfindungsgemäß**e**n Proteingemisch, Vektor oder Zelle wird jede Möglichkeit der Wechselwirkung zwischen den beiden Komponenten verstanden, wobei sich die beiden Komponenten jeweils unabhängig voneinander in flüssiger Phase, beispielsweise in Lösung oder in einer Suspension, befinden können oder auch an eine feste Phase, beispielsweise in Form einer im wesentlichen planaren Oberfläche oder in Form von Partikeln, Perlen oder ähnlichem, gebunden sein können. In einer bevorzugten Ausführungsform ist eine Vielzahl unterschiedlicher potentiell bindender Substanzen an einer festen Oberfläche immobilisiert und wird mit den erfindungsgemäßen Proteingemisch, dem erfindungsgemäßen Vektor oder der erfindungsgemäßen Zelle In-Kontakt gebracht und anschließend wird die Bindung der erfindungsgemäßen Substanzen an den verschiedenen Positionen, an denen jeweils unterschiedliche potentiell-bindende Substanzen immobilisiert sind, gemessen.

Das Messen der Bindung des erfindungsgemäßen Proteingemisches, des Vektors oder der Zelle, an eine potentiell bindende Substanz kann beispielsweise über die Messung eines mit dem erfindungsgemäßen Proteingemisch, mit dem erfindungsgemäßen Vektor oder der erfindungsgemäßen Zelle verbundenen Markers geschehen, wobei geeignete Marker dem Fachmann bekannt sind und beispielsweise Fluoreszenz- oder radioaktive Marker umfassen. In einer bevorzugten Ausführungsform enthält das Proteingemisch, der Vektor oder die Zelle zusätzlich im zweiten Fusionsprotein neben dem Protein oder Proteinfragment, dessen Wechselwirkung mit den potentiell-bindenden Substanzen untersucht werden soll, ein autofluorezierendes Protein, wie beispielsweise GFP oder Varianten davon. Das Messen der Bindung der Substanz kann jedoch auch über die Änderung von elektrochemischen, insbesondere Redoxeigenschaften beispielsweise der immobilisierten potentiell bindenden Substanzen nach In-Kontaktbringen gemessen werden. Geeignete Verfahren umfassen beispielsweise potentiometrische Methoden. Weitere Verfahren zum Nachweis der Bindung zweier Moleküle oder Molekülgemische, sind dem Fachmann bekannt und können gleichermaßen zum Messen der Bindung der potentiell-bindenden Substanz an das erfindungsgemäße Proteingemisch, den erfindungsgemäßen Vektor oder die erfindungsgemäße Zelle verwendet werden.

Gegebenenfalls können vor, zwischen oder nach den Schritten des erfindungsgemäßen Verfahrens weitere Schritte eingeführt werden, wie beispielsweise das ein- oder mehrmalige Waschen nach dem In-Kontaktbringen, um beispielsweise unspezifische Bindungen zwischen der potentiell bindenden Substanz und dem erfindungsgemäßen Proteingemisch, dem erfindungsgemäßen Vektor oder der erfindungsgemäßen Zelle zu lösen.

Als weitere Schritte nach dem Messen der Bindung der Substanz kann eine bindende Substanz z.B. auf Grund der gemessenen Bindungsstärke ausgewählt werden und dann direkt bspw. zur Inhibition des bekannten Proteintargets verwendet werden. Die bindende Substanz kann jedoch auch durch im Stand der Technik bekannte Verfahren, die auch Verfahren der kombinatorischen Chemie umfassen modifiziert werden. Beispielsweise durch das Anfügen von Halogenseitengruppen, vorzugsweise F oder Cl, durch das Anfügen von nieder Alkylgruppen, wie Methyl-, Ethyl-, n-Propyl-, iso-Propyl-, n-Butyl-, iso-Butyl- oder tert-Butylgruppen oder durch das Anfügen von Amino-, Nitro-, Hydroxyl-, Amido- oder Carbonsäuregruppen. Die so unterschiedlich modifizierten bindenden Substanzen können dann erneut in dem erfindungsgemäßen Verfahren auf ihre Bindung getestet werden und hinsichtlich der gewünschten Bindungsspezifität und dem dadurch erzielten Effekt (beispielsweise Aktivierung, Inhibierung oder Modulation der jeweiligen Aktivität) optimiert werden.

Ein weiterer Gegenstand der vorliegenden Erfindung ist ein Verfahren zum Auffinden von Proteinen oder Proteinfragmenten, die an eine Testsubstanz binden, enthaltend die Schritte:
a) In-Kontaktbringen mindestens einer Testsubstanz mit einer erfindungsgemäßen Bibliothek und
b) Messen der jeweiligen Bindung der Testsubstanz an die unterschiedlichen Proteingemische, Vektoren und/oder Zellen der erfindungsgemäßen Bibliothek.

Bei diesem Verfahren sollen Proteine oder Proteinfragmente ausgewählt werden, die an eine gegebene Testsubstanz binden. Vorzugsweise handelt es sich dabei um die Proteine oder Proteinfragmente des zweiten Fusionsproteins, da diese mit einer größeren Wahrscheinlichkeit korrekt gefaltet sind, als die Proteine oder Proteinfragmente des ersten Fusionsproteins, die nur dann korrekt gefaltet sind, wenn die jeweiligen Proteine auch im oxidativen Mileu des Periplasmas ihre native Konformation annehmen können. Eine Testsubstanz im Sinne der vorliegenden Erfindung kann jede beliebige chemische Substanz oder ein Gemisch davon sein. Vorzugsweise handelt es sich dabei jedoch um ein Protein oder Proteinfragment, insbesondere um einen Rezeptor, einen Rezeptorliganden, einen Transkriptionsfaktor, einen Ionenkanal, ein Molekül der Signaltransduktionskaskade, Struktur- und Speicherprotein, Toxin oder Lichtrezeptor- und Pigmentprotein. Das Messen der jeweiligen Bindung der unterschiedlichen Proteingemische, Vektoren und/oder Zellen der Bibliothek an die Testsubstanz kann wie vorangehend beschrieben, über- markierungsabhängige oder markierungsunabhähngige Meßverfahren erfolgen.

In einer bevorzugten Ausführungsform des erfindungsgemäßen Verfahrens enthält das Verfahren die weiteren Schritte: Auswählen mindestens eines Proteingemisches, eines Vektors oder einer Zelle aufgrund der gemessenen Bindung und Herstellen einer zweiten Bibliothek, wobei die Bibliothek durch Modifikation des im ausgewählten Proteingemisch, im ausgewählten Vektor oder in der ausgewählten Zelle enthaltenen Proteins oder Proteinfragments erzeugt wird. Der Auswahlprozeß von Proteingemischen, Vektoren oder Zellen aus der Bibliothek wird vorzugsweise aufgrund der Stärke der Bindung durchgeführt, wobei die Proteingemische, Vektoren oder Zellen bevorzugt sind, die die stärkste Bindung an die jeweilige Testsubstanz zeigen. Ausgehend von der Aminosäuresequenz des im ausgewählten Proteingemisch, Vektor oder Zelle enthaltenen Proteins oder Proteinfragments, die durch Standardverfahren bestimmt werden kann, können Modifikation erzeugt werden, die jeweils zu geringen Änderungen der Aminosäuresequenz führen und damit zu einer Vielzahl von Derivaten, die im Vergleich zum Ausgangsprotein bzw. Proteinfragment eine leicht veränderte dreidimensionale Struktur besitzen. Solche Modifikationen lassen sich durch im Stand der Technik bekannte Verfahren beispielsweise durch Zufallsmutagenese oder auch durch gezielte Substitution einzelner Nukleinsäurecodons, der für das Protein oder Proteinfragment kodierenden Nukleinsäure, erhalten. Bevorzugt sind dabei Substitution, die sogenannte "konservative" Substitutionen sind. Eine konservative Substitution liegt dann vor, wenn beispielsweise ein für eine basische Aminosäure kodierendes Nukleinsäurecodon durch ein anderes für eine basische Aminosäure kodierendes Nukleinsäurecodon, ein für eine saure Aminosäure kodierendes Nukleinsäurecodon durch ein anderes für eine saure Aminosäure kodierendes Nukleinsäurecodon bzw. ein für eine polare Aminosäure kodierendes Nukleinsäurecodon durch ein anderes für eine polare Aminosäure kodierendes Nukleinsäurecodon ersetzt wird.

Die auf Grundlage der ausgewählten Proteingemische, Vektoren oder Zellen neu erzeugten zweiten Bibliotheken können nunmehr in einem weiteren Schritt wiederum mit der Testsubstanz in Kontakt gebracht werden, worauf in einem weiteren Schritt die jeweilige Bindung der Testsubstanz an die modifizierten Proteingemische, Vektoren oder Zellen der zweiten Bibliothek gemessen werden kann. Gegebenenfalls können nunmehr die Schritte des Auswählens mindestens eines Proteingemisches, eines Vektors oder einer Zelle aufgrund der gemessenen Bindung und die sich daran anschließend Herstellung einer dritten bzw. n-ten Bibliothek sowie das Inkontaktbringen und Messen der jeweiligen Bindung der Testsubstanz an die unterschiedlichen Proteingemische, Vektoren oder Zellen der dritten bzw. n-ten Bibliothek, ein bis n-mal wiederholt werden bis ein Proteingemisch, ein Vektor oder eine Zelle ausgewählt worden ist, das, der bzw. die die gewünschte Bindung zeigt.

Das zuvor geschilderte Verfahren wird auch als gerichtete Evolution bezeichnet, da in einer Vielzahl von Schritten die aus Modifikation und Selektion bestehen, Proteine oder Proteinfragmente "evolutionär" hinsichtlich einer bestimmten Eigenschaft, insbesondere ihrer Bindungseigenschaft weiterentwickelt werden.

Die durch das obige Verfahren aufgefundenen oder zusätzlich hinsichtlich einer bestimmten Eigenschaft optimierten Proteine oder Proteinfragmente können, wenn sie beispielsweise auf die Aktivierung oder Repression eines bestimmten zellulären Signalwegs hin optimiert worden sind, als Wirkstoff in einem Medikament eingesetzt werden. Dasselbe gilt für die bindenden Substanzen, die in dem Verfahren zum Auffinden von potentiell bindenden Substanzen identifiziert worden sind. Daher umfassen die erfindungsgemäßen Verfahren in einer bevorzugten Ausführungsform den weiteren Schritt, daß die ausgewählte bindende Substanz oder das in dem ausgewählten Proteingemisch, in dem ausgewählten Vektor oder in der ausgewählten Zelle enthaltene Protein oder Proteinfragment oder eine Variante davon mit einem pharmazeutisch akzeptablem Träger und/oder Hilfsstoff gemischt wird.

Eine "Variante" des Proteins oder Proteinfragments beinhaltet Modifikation des N- oder C-Terminus oder Modifikation von Aminosäureseitenketten, die beispielsweise die Stabilität, Löslichkeit oder Biokompatibilität des Proteins oder Proteinfragments erhöhen. Umfaßt sind jedoch auch Fusionsproteine mit den erfindungsgemäß identifizierten Proteinen oder Proteinfragmenten, die als weitere Komponenten beispielsweise autofluoreszente Marker, wie beispielsweise GFP oder Zytostatika wie beispielsweise Choleratoxin enthalten können.

Pharmazeutisch akzeptable Träger und/oder Hilfsstoffe umfassen Substanzen, die die bindende Substanz bzw. das Protein oder Proteinfragment oder seine Varianten stabilisieren, deren pharmazeutische Verträglichkeit erhöhen oder die für die jeweilige Applikationsform, wie beispielsweise Tablette, Pflaster oder Infusionslösung, erforderlich sind, wie beispielsweise Konservierungsmittel, Puffer, Salz oder Proteaseinhibitoren.

Ein weiterer Gegenstand der vorliegenden-Erfindung ist ein Kit zur Herstellung eines Nukleinsäuregemisches nach Anspruch 10 enthaltend:
a) mindestens eine erste Nukleinsäure enthaltend mindestens eine Restriktionsschnittstelle 5' und/oder 3` von einer Nukleinsäure kodierend für ein erstes Fusionsprotein enthaltend:
   i) eine Interaktionsdomäne und
   ii) eine Proteintranslokationssequenz, die bewirkt, daß das erste Fusionsprotein bei Expression in einem Bakterium in einem im wesentlichen gefalteten Zustand durch die zytoplasmatische Membran transloziert wird.

Dieses Kit erlaubt die Insertion einer ausgewählten Nukleinsäuresequenz 5' oder 3' von der Nukleinsäure, die für eine Interaktionsdomäne und eine Proteintranslokationssequenz kodiert, so daß im Ergebnis von der resultierenden Nukleinsäure ein Fusionsprotein kodiert wird, das am C-Terminus und/oder am N-Terminus ein durch die jeweils eingeführte Nukleinsäuresequenz kodiertes Protein oder Proteinfragment enthält. Vorzugsweise handelt es sich bei der eingeführten DNA um eine cDNA-Bibliothek, wobei diese besonders bevorzugt unter Verwendung der 3'-Restriktionsschnittstelle in die Nukleinsäure eingeführt wird. In einer bevorzugten Ausführungsform enthält das Kit den Leuzin-Zipper aus dem cFos-Protein und in einer weiteren Bevorzugten Ausführungsform die Tat-abhängige Proteintranslokationssequenz TorA.

In einer weiteren Ausführungsform des erfindungsgemäßen Kits enthält das Kit des weiteren mindestens eine zweite Nukleinsäure enthaltend mindestens eine Restriktionsschnittstelle 5' und/oder 3' von einer Nukleinsäure kodierend für ein zweites Fusionsprotein enthaltend:
i) eine Interaktionsdomäne und
ii) eine Proteintranslokationssequenz, die bewirkt, daß das zweite Fusionsprotein bei Expression in einem Bakterium in einem im wesentlichen ungefalteten Zustand durch die zytoplasmatische Membran transloziert wird, wobei die Interaktionsdomäne des ersten Fusionsproteins an die des zweiten Fusionsproteins binden kann.

Diese Nukleinsäure erlaubt die Insertion 5' oder 3` von der für eine Interaktionsdomäne und eine Proteintranslokationssequenz kodierenden Nukleinsäure, so daß im Ergebnis von der resultierenden Nukleinsäure ein Fusionsprotein kodiert wird, das am N- oder C-Terminus ein von der insertierten Nukleinsäure kodiertes Protein oder Proteinfragment umfaßt. Beispielsweise können Nukleinsäuren, die für Phagenhüllproteine kodieren in die Nukleinsäure insertiert werden, wobei diese vorzugsweise in die 3'-Restriktionsschnittstelle eingeführt werden.

Es hat sich gezeigt, daß wenn in die zweite Nukleinsäure Nukleinsäuren eingeführt werden, die für Phagenhüllproteine kodieren, daß die resultierenden Fusionsproteine bei starker Expression von beispielsweise dem gIIIp-Fusionsprotein zu eine hohen Toxizität für *E*. *coli-*Zellen führen. Aus diesem Grund wird bei klassischen Phagendisplaysystemen ein Amber-Codon 5' von dem gIII-Protein eingefügt. In Supressorstämmen (z.B. XL-1 Blue) wird dadurch die Expression des gIIIp-Fusionsproteins um ca. 90% reduziert. Darüber hinaus ermöglicht das Amber-Codon (das in Nicht-Suppressorstämmen als STOP-Codon gelesen wird) sehr einfach die lösliche Expression des zuvor mit dem Phagenprotein fusionierten und auf dem Phagen präsentierten Proteins, indem das Phagemid in einen Nicht-Suppressorstamm (z.B. BL21) eingebracht und dort die Expression durchgeführt wird. Daher enthält die erste und/oder die zweite Nukleinsäure in einer bevorzugten Ausführungsform entweder 5 oder 3' ein Amber-Codon. Vorzugsweise ist das Amber-Codon in der ersten Nukleinsäure 5' angeordnet und in der zweiten Nukleinsäure 3` angeordnet. Dadurch läßt sich in einem geeigneten bakteriellen Wirt erreichen, daß ausschließlich das in der ersten Nukleinsäure 5'eingefügte Protein oder Proteinfragment exprimiert wird und gleichzeitig der toxische Effekt des in der zweiten Nukleinsäure 3' eingefügten gIIIp verhindert wird.

In einer weiteren bevorzugten Ausführungsform des erfindungsgemäßen Kits ist die Interaktionsdomäne des zweiten Fusionsproteins die Leuzin-Zipperdomäne des cJun-Proteins. In einer weiteren bevorzugten Ausführungsform enthält die Nukleinsäure eine Nukleinsäure, die für eine Sec-abhängige Proteintranslokationssequenz insbesondere das PelB-Führungspeptid kodiert.

Ein weiterer Gegenstand der vorliegenden Erfindung ist die Verwendung einer Zelle zur Herstellung eines erfindungsgemäßen Proteingemisches sowie die Verwendung eines erfindungsgemäßen Proteingemisches, eines erfindungsgemäßen Vektors oder einer erfindungsgemäßen Zelle zur Herstellung einer erfindungsgemäßen Bibliothek.

Ein bevorzugtes Anwendungsgebiete der erfindungsgemäßen Proteingemische, erfindungsgemäßen Phagen, der erfindungsgemäßen Zellen, insbesondere der erfindungsgemäßen Bibliotheken, enthaltend vorgenannte Proteingemische, Phagen und Zellen sowie der erfindungsgemäßen Kits, ist die Präsentation von Proteinen auf filamentösen Phagen. Ein besonderer Schwerpunkt liegt dabei auf Proteinen, die aufgrund ihrer Inkompatibilität mit dem Sec-Transportweg nicht mit Hilfe der klassischen Phagendisplay-Technologie präsentiert werden können. Als besonders bevorzugte Anwendungsbereiche ergeben sich daher die Präsentation und Selektion von cDNA-Expressionsbibliotheken und die Präsentation und Selektion von DNA-Bibliotheken zur gerichteten Evolution von Proteinen, auch "protein engineering" genannt.

Eine weitere bevorzugte Verwendung liegt in der Herstellung von Proteinkonjugaten. Dabei ist die Verwendung dann besonders bevorzugt, wenn das Protein oder Proteinfragment des ersten Fusionsproteins und das Protein oder Proteinfragment des zweiten Fusionsproteins jeweils unterschiedliche Anforderungen hinsichtlich der zur korrekten Faltung erforderlichen zellulären Umgebung haben. So erlaubt es die vorliegende Erfindung Antikörper direkt mit Markerproteinen gentechnisch zu fusionieren, die bei Produktion in Bakterien und Transport über den Sec-abhängigen Transportweg nicht korrekt gefaltet würden und daher bei Anwendung von Standardverfahren nicht als Markerprotein zur Markierung des Antikörper verwendet werden können. Markerproteine-Antikörperfusion, deren funktionelle Expression erst durch die vorliegende Erfindung möglich geworden ist, umfassen beispielsweise Fusionen aus autofloreszierenden Proteine, wie GFP und schweren Immunoglobulinketten, leichten Immunoglobulinketten oder "single-chain-antibodies".

### Abbildungen

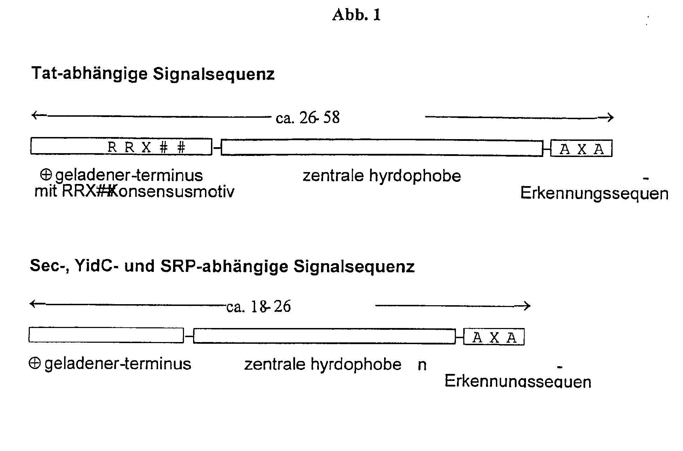

- Abb. 1: Konsensussequenzen Tat-abhängiger, Sec-abhängiger, SRP-abhängiger oder YidC-abhängiger Signalsequenzen, wobei X eine beliebige Aminosäure und # eine hydrophobe Aminosäure präsentieren.

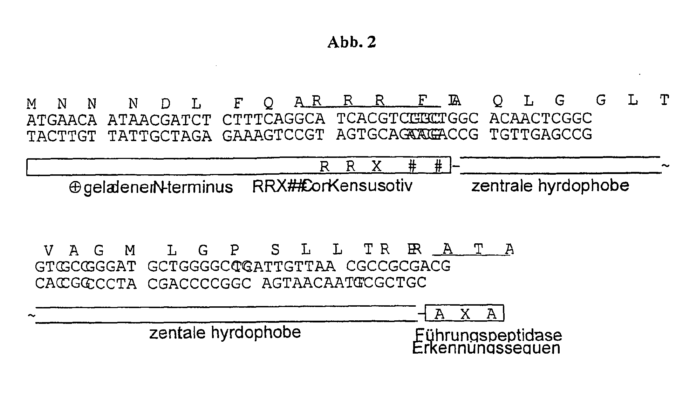

- Abb. 2: Tat-abhängiges TorA-Signalpeptid, wobei X eine beliebige Aminosäure und # eine hydrophobe Aminosäure präsentieren

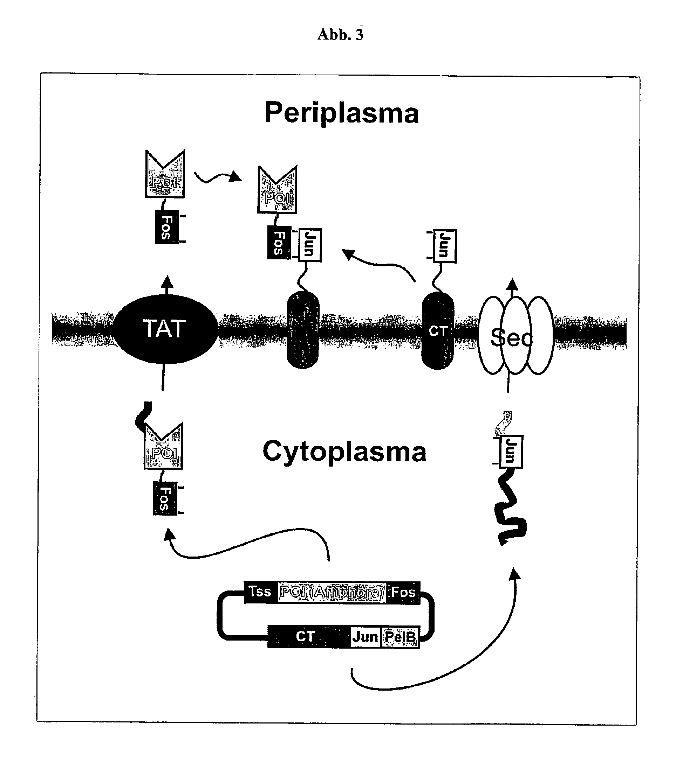

- Abb. 3: Prinzip des TLF-Systems, wobei CT die Domäne des pIII, pelB die Sec-Signalsequenz, TSS die Tat-Signalsequenz und POI das präsentierte Protein bedeuten.

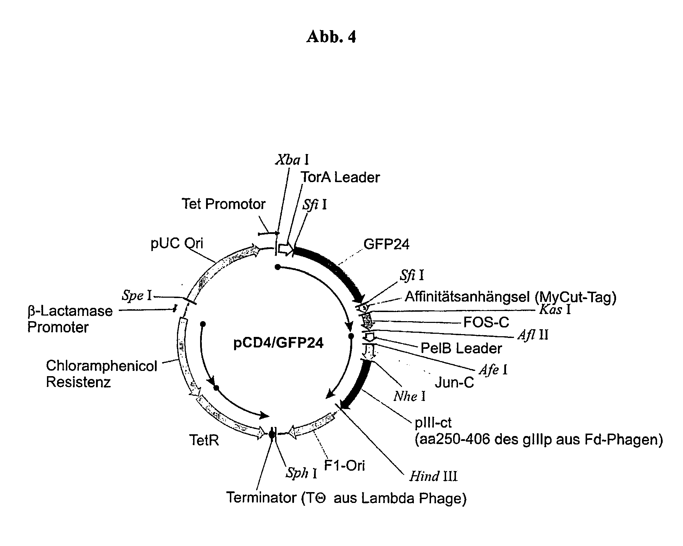

- Abb. 4: Restriktionskarte des Plasmids pCD4/GFP24, dessen Nukleinsäuresequenz als SEQ ID NR: 1 im Anhang wiedergegeben ist.

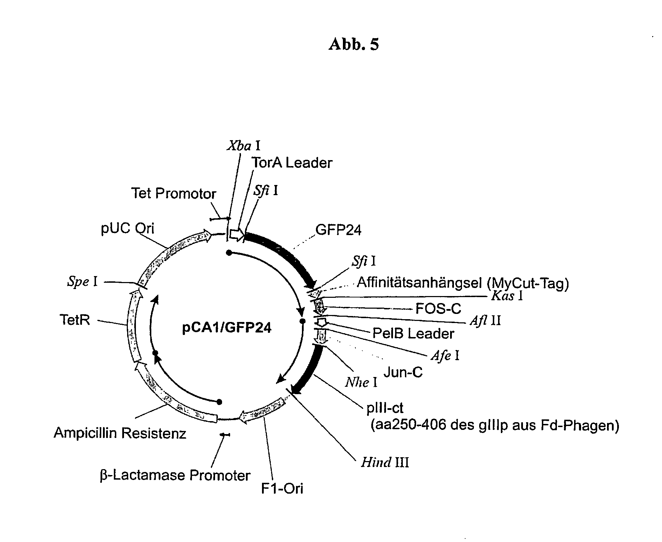

- Abb. 5: Restriktionskarte des Plasmids pCA1/GFP24, dessen Nukleinsäuresequenz in SEQ ID NR: 2 wiedergegeben ist.

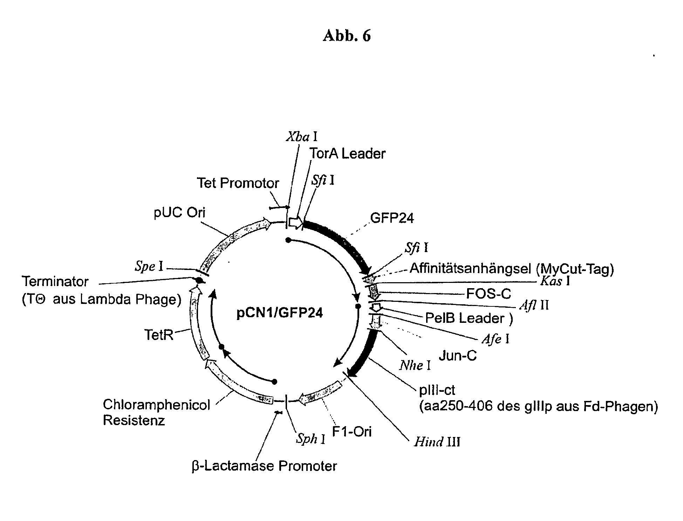

- Abb. 6: Restriktionskarte des Plasmids pCN1/GFP24, dessen Nukleinsäuresequenz in SEQ ID NR: 3 wiedergegeben ist.
- Abb. 7: Kompetitiver Phagen-ELISA, wobei weiße Balken die Ergebnisse mit GFP24-präsentierende Phagen zeigen. GFP24-Phagen wurden mit Hilfe von XL-1 Blue Zelle hergestellt, die das Plasmid pCD4/GFP24 tragen. Graue Balken präsentieren die Ergebnisse, die mit β-Lactamase-tragenden Phagen erzielt wurden. β-Lactamase-präsentierende Phagen wurden in XL-1 Blue Zelle hergestellt, die das Plasmid pCD4BLA trugen.
- Abb. 8: Enzymatischer Assay, der Präsentation von β-Lactamase auf Bakteriophage, wobei weißen Kreise die Ergebnisse mit GFP24-tragende Phagen zeigen. GFP24-Phagen wurden mit Hilfe von XL-1 Blue Zelle hergestellt wurden, die das Plasmid pCD4/GFP24 tragen. Schwarze Vierecke präsentieren die Ergebnisse die mit β-Lactamase-tragenden Phagen erzielt wurden. β-Lactamase-präsentierende Phagen wurden in XL-1 Blue Zelle hergestellt, die das Plasmid pCD4BLA trugen. Gezeigt wird die Absorption bei 486 nm in Abhängigkeit von der Zeit.

### Beispiele

### Beispiel 1: Verwendete Vektoren

pCD4/GFP24 ist ein Cysteindisplay-Phagemid, das auf dem pGP-Vektor (Paschke M., et al.: (2001) Biotechniques 30: 720-725) beruht.

pCAl/GFP24 ist ein Cysteindisplay-Phagemid, das auf pGP-F100 basiert. Es kann für die tet^{0-p}-kontrollierte Expression von Protein als Fusion von cFos-Leuzin-Zipper verwendet werden. Die Translokation des cFos-Fusionsproteins im periplasmatischen Raum wird durch die TorA-Führungspeptidsequenz (Tat-abhängige Translokationsweg) vermittelt. Das tet^{0-p}-kontrollierte Transkript enthält ein zweites Cistron, von dem das c-jun::G3Ps-Fusionsprotein exprimiert wird. Das c-Jun::G3Ps wird über den Sec-abhängigen Translokationsweg in dem periplasmatischen Raum gesteuert. Kovalente Komplexe zwischen dem cFos-Fusionsprotein und dem cJun::G3PS-Fusionsprotein werden im periplasmatischen Raum aufgrund der Dimerisierung von cJun und cFos und anschließender Ausbildung von Cysteinbindungen zwischen den Proteinen hergestellt. Das Phagemid enthält eine GFP24-Kassette, flankiert von Sfil-Restriktionsstellen an den Positionen 148 und 910 und ist zwischen dem TorA-Führungspeptid und cFos angeordnet. Diese Kassette muß durch das zu präsentierende Protein ersetzt werden.

pCAl/GFP24 ist ein von pCD4 abgeleitetes Cysteindisplay-Phagemid, das auf dem pGP-Vektor basiert. pCD4/GFP24 ist ein Cysteindisplay-Phagnid, das auf dem pGP-Vektor (Paschke M., et al.: (2001) Biotechniques 30: 720-725) beruht. Es kann für die tet^{o-p}-kontrollierte Expression von Proteinen als Fusion mit dem cFos-Leucinzipper verwendet werden. Die Translokation des cFos-Fusionsproteins in den periplasmatischen Raum wird durch das TorA-Führungspeptid (Tat-Transportweg) vermittelt. Das tet^{o-p}-kontrollierte Transkript enthält ein zweites Cistron, von dem das c-jun::G3Pss Fusionsprotein exprimiert wird (G3Pss umfaßt Aminosäuren 252 bis 406 des reifen gIII-Proteins des fd-Phagen). Das c-jun:G3Pss wird durch einen Sec-abhängigen Transportweg (pelB-Führungspeptid) in dem periplasmatischen Raum gelenkt. Kovalente Komplexe von cFos-Fusionsprotein und c-jun::G3Pss werden aufgrund der Dimerisierung zwischen cJun und cFos im periplasmatischen Raum und die anschließende Ausbildung von Cysteinbindung zwischen den Proteinen gebildet (Crameri, R. und Suter M. (1993), siehe oben). Der Phagendisplay der Proteine, die mit cFos fusioniert sind, kann durch sogenannten Helferphargenrescue erreicht werden. Im Gegensatz zu pGP überträgt das Phagemid pCD4-GFP24 Chloramphenicolresistenz. Das Resistenzgen (CAT) und der tet-Repressor (TetR) werden unter Kontrolle des β-Lactamasepromotors von einer bicistronischen Kassette kontrolliert. Das Transkript wird in einem λ-Phagenterminator terminiert. Die Tat-TetR-Kassette ist in umgekehrter Orientierung zu der cFos- und cJun-Fusionskassette. Eine GFP24-Kassette, flankiert an der Position 148 und 910 von SfiI-Restriktionsstellen ist zwischen dem TorA-Führungspeptid und cFos angeordnet. Diese Kassette wird durch das zu präsentierenden Protein ersetzt.

pCD4Bla ist ein von pCD4/GFP24 abgeleitetes Cysteindisplay-Phagemid, bei dem mittels Restriktion mit Sfil das GFP24 Fragment durch die Sequenz der reifen TEM1 β-Lactamase ersetzt wurde. Die eingefügte Lactamaseklonierungskasette mit 5' und 3'-terminalen Sfil-Restriktionsstellen ist in SEQ ID NR: 4 wiedergegeben.

### Beispiel 2: Herstellung von Bakteriophagen

Mit dem entsprechenden Phagemid transformierte XLI-Blue-Zellen wurden in 2 TY-Selektionsmedium bei 30°C bis zu einem OD₆₀₀ₙₘ, von 0,5 kultiviert und dann mit dem Helferphagen VCSM13 mit einem Moi = 10-20 gemischt. Die infizierte Kultur wurde für 30 Minuten bei 37°C kultiviert und anschließend mit Kanamizin in einer Endkonzentration von 60 ug/ml versetzt. Die Kultur wurde für 10 Minuten bei 25°C kultiviert. Durch Zentrifugation (4000 x g, 4°C, 5 Minuten) wurden die Zellen geerntet und anschließend in 2 TY-Selektionsmedium enthaltend 60 µg/l Kanamizin und 0,5 µg/ml Tetracyclin resuspendiert. Diese Kultur wurde für 5 h bei 25°C kultiviert. Anschließend folgte die Phagenpräparation aus dem Zellkulturüberstand wie folgt: Jeweils 40-50 ml Zellen und Zelltrümmer wurden durch Zentrifugation (4°C, 10.000 rpm in einem A8-24-Rotor für 15 Minuten) vom phagenhaltigen Zellkulturüberstand getrennt. Der Überstand wurde durch einen 0,45 µm Filter filtriert und mit 1/4 Volumen PEG-NaCl-Lösung (20% w/v PEG 8000, 50% w/v NaCl) gemischt und über Nacht oder für mindestens eine Stunde auf Eis inkubiert. Die Mischung wurde dann bei 4°C für 15 min bei 15.000 rpm in einem A8.24-Rotor zentrifugiert. Der Niederschlag wurde in 2,5 ml eiskaltem PBS resuspendiert und auf 2 ml Plastikröhrchen verteilt. Dann wurde der Überstand mit 1/4 Volumen PEG-NaCl-Lösung gemischt und für mindestens eine weitere Stunde auf Eis inkubiert. Anschließend wurde der Überstand bei 4°C und 14.000 rpm für 15 Minuten zentrifugiert. Der Phagenniederschlag wurde in 0,5-1 ml PBS gelöst. Gegebenenfalls wurde die Phagenlösung durch einen 0,45 µm-Filter filtriert und anschließend bei 4°C gelagert. Bei längerer Lagerung wurde die Phagenlösung mit 1 Volumen Glycerol versetzt und bei -70°C gelagert.

Der Phagentiter wurde durch Standardverfahren unter Verwendung einer Verdünnungsreihe bestimmt. Der Titer lag üblicherweise zwischen 10¹² und 10¹³ cfu/ml.

### Beispiel 3: Präsentation von funktionellem GFP24 auf Phagen

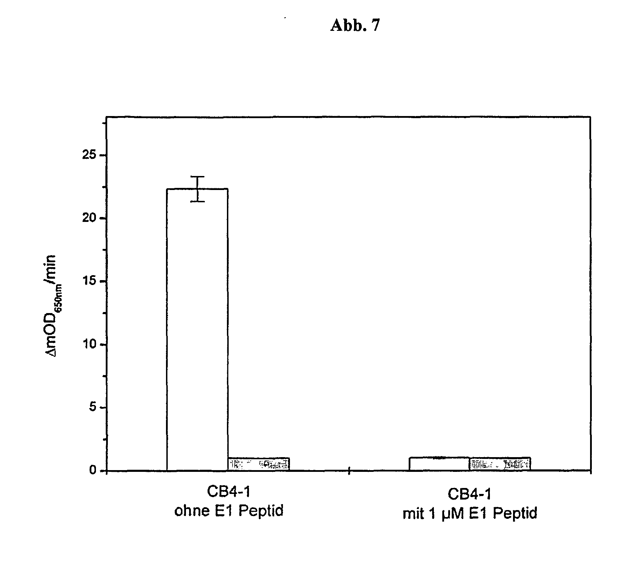

GFP24 ist eine zirkulärpermutierte Variante des grün fluoreszierenden Proteins, die zusätzlich ein Epitop des P24-Proteins aus HIV enthält (Höhne, W.E. et al. (1993) Mol Immunol 30:1213-21). GFP24 wird durch den anti-P24-Antikörper CB4-1 (Dr. Scholz, Institut für Biochemie, (Universitätsklinikum Charite)) mit hoher Affinität gebunden. GFP24 kann wie auch GFP selbst nicht über den Sec-Transportweg exportiert werden. Ein funktionelles GFP24 Protein sollte daher bei Expression des oben geschriebenen pCD4/GFP24-Plasmids nur dann zur Präsentation eines funktionsfähigen GFP24 führen, wenn dieser Teil des Proteins nicht durch einen Sec-abhängigen Transportweg, sondern durch einen Tat-abhängigen Transportweg in das Periplasma transportiert worden ist. Um GFP24 auf filamentösen Phagen nachzuweisen, wurde ein Phagen-ELISA wie folgt durchgeführt. Mikrotiterplatten wurden mit 10 µm/ml anit-P24Antikörper-C4-1 beschichtet, dreimal mit PBS/Tween 0,1% gewaschen und mit 200 µl pro Vertiefung Genosys-Blockierungsreagenz (Sigma-Genosys Ltd, Cambridge, UK)) für ein bis zwei Stunden bei Raumtemperatur unter Schütteln inkubiert. Anschließend wurde die Mikrotiterplatte dreimal mit PBS/Tween 20 0,1% gewaschen. Anschließend wurden 50 µl pro Vertiefung GFP24 präsentierende Phagen mit und ohne P24-Peptid in die Mikrotiterplatte gegeben und anschließend die Gegenwart des Phagen in der Mikrotiterplatte mit einem Meerrettichperoxidase gekoppelten anti-Phagen-Antikörper (Seramun Diagnostica GmbH, Dolgenbrodt, Germany)) nachgewiesen. Die Signalstärke entsprach dabei dem an CB4-1 gebundenen Phagen. pCD4/GFP24 Phagen wurden dabei vollständig von CB4-1 durch das P24-Peptid verdrängt, während β- Lactamase präsentierende Phagen (pCD4/BLA), die als Kontrolle verwendet wurden, nicht an CB4-1 gebunden wurden. Darüber hinaus konnte keine unspezifische Bindung an anderer Antikörper oder das Blockierungsreagenz beobachtet werden (s. Abb. 7).

### Beispiel 4: Präsentation von TEM-1-β- Lactamase auf filamentösen Phagen

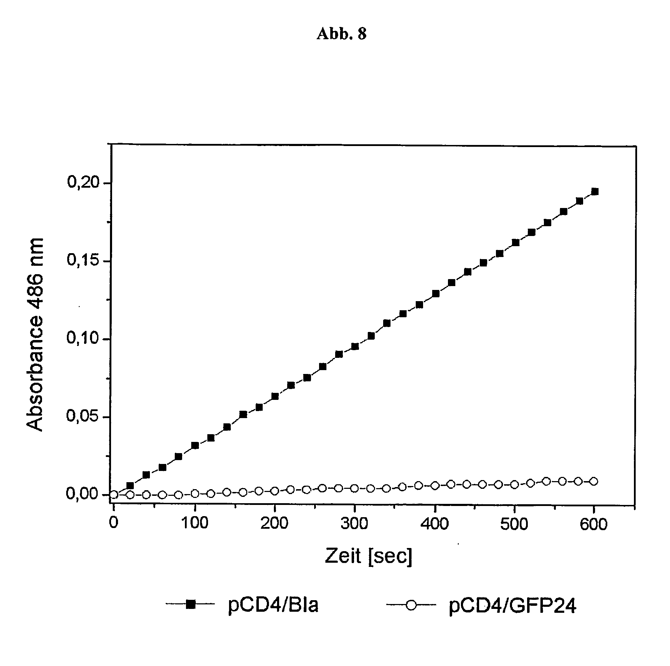

TEM-1-β-Lactamase ist ein periplasmatischen Protein, das durch die Hydrolyse des Lactamrings des Antibiotikums Ampicillin Resistenz gegen Ampicillin vermitteln kann. TEM-β-Lactamase wird normalerweise mittels des Sec-abhängigen Transportsystems ins Periplasma exportiert. Zum Nachweis, daß TEM-1-β- Lactamase auch durch den Tat-abhängigen Transportweg exportiert werden kann, wurde die Sec-Signalsquenze entfernt und durch die TorA-Sequenz ersetzt. Die erfolgreiche Präsentation von TEM-1-β- Lactamase wurde mit dem nachfolgend beschriebenen Enzymessay nachgewiesen, dessen Ergebnis in Abb. 8 dargestellt ist. 800 µl PBS pH 7,4 wurden mit 100 µl Nitrocefin-Stammlösung (500 ug/ml) gemischt und auf 25°C temperiert. 100 µl Phagenlösung wurden hinzugegeben. Die Extinktsionsänderung bei 486 nm wurde photometrisch über 10 min. beobachtet. Die Absorptionsänderung bei 486 nm entspricht dabei der β-Lactamaseaktivität der Phagen. Während pCD4/GFP24-Phagen keine β-Lactamaseaktivität zeigten, war bei pCD4/BLA eine starke β-Lactamaseaktivität zu beobachten.

Die Nitrocefinstammlösung wurde folgendermaßen hergestellt: 1 mg Nitrocefin wurd in 100 µl DMSO gelöst. Diese Lösung wurde dann mit 1,9 ml PBS pH 7,4 gemischt. Die Lösung wurde für maximal zwei Wochen bei -20°C gelagert.

## Patentansprüche

1. Proteingemisch enthaltend:
a) mindestens ein erstes Fusionsprotein enthaltend:
i) ein Protein oder Proteinfragment,
ii) eine Interaktionsdomäne und
iii) eine Proteintranslokationssequenz, die bewirkt, daß das Fusionsprotein bei Expression in einem Bakterium in einem im wesentlichen ungefalteten Zustand durch die zytoplasmatische Membran transloziert wird,
und
b) mindestens ein zweites Fusionsprotein enthaltend:
i) ein Protein oder Proteinfragment,
ii) eine Interaktionsdomäne und
iii) eine Proteintranslokationssequenz, die bewirkt, daß das Fusionsprotein bei Expression in einem Bakterium in einem im wesentlichen gefalteten Zustand durch die zytoplasmatische Membran transloziert wird,
wobei die Interaktionsdomäne des ersten Fusionsproteins an die des zweiten Fusionsproteins binden kann.

2. Proteingemisch nach Anspruch 1, wobei das Protein oder Proteinfragment des ersten Fusionsproteins eine schwere Immunoglobulinkette, eine leichte Immunoglobulinkette, ein single-chain Antikörper, ein Diabody, ein Rezeptor, ein Rezeptorligand, ein Integrin, ein Intimin, ein Kohlenhydrat-bindendes Protein, ein Albumin-bindendes Protein oder Protein A ist.

3. Proteingemisch nach Anspruch 1 oder 2, wobei das Protein oder Proteinfragment des zweiten Fusionsproteins ein autofluoreszierendes Protein, insbesondere GFP oder eine Variante davon, ein Enzym, ein Cofaktor-abhängiges Protein, ein Protein, das von einer aus einer cDNA-Bibliothek stammenden cDNA kodiert wird, oder ein synthetisches Protein ist.

4. Proteingemisch nach Anspruch 1, wobei das Protein oder Proteinfragment des ersten Fusionsproteins und die Proteintranslokationssequenz ein Phagenhüllprotein, ein periplasmatisches Markerenzym, ein Intimin, ein Protein der äußeren Bakterienmembran oder ein periplasmatisches Rezeptorprotein ist.

5. Proteingemisch nach Anspruch 4, wobei das Phagenhüllprotein ausgewählt ist aus den M13-Phagenhüllproteinen pIII, pVI, pVII, pVIII und pIX.

6. Proteingemisch nach einem der Ansprüche 1 bis 5, wobei die Interaktionsdomänen des ersten und zweiten Fusionsproteins jeweils eine Leuzin-Zipper-Domäne und eine Leuzin-Zipper-Domäne, eine Helix-Loop-Helix-Domäne und eine Helix-Loop-Helix-Domäne, ein Calmodulin und ein Calmodulin-bindendes Peptid oder ein Peptid-Dimer-Paar natürlichen oder synthetischen Ursprungs sind.

7. Proteingemisch nach einem der Ansprüche 1 bis 6, wobei die Proteintranslokationssequenz des ersten Fusionsproteins eine Sec-abhängige, SRP-abhängige, YidCabhängige Sequenz oder eine Transportweg-unabhängige Sequenz, die in die Membran integriert wird, ist.

8. Proteingemisch nach einem der Ansprüche 1 bis 7, wobei die Proteintranslokationssequenz des zweiten Fusionsproteins eine Tat-abhängige oder Thylakoid-ApHabhängige Sequenz ist.

9. Proteingemisch nach einem der Ansprüche 1 bis 8, wobei das erste Fusionsprotein an das zweite Fusionsprotein kovalent oder nicht-kovalent gebunden ist.

10. Nukleinsäuregemisch kodierend für ein Proteingemisch nach einem der Ansprüche 1 bis 8.

11. Nukleinsäuregemisch nach Anspruch 10, wobei mindestens zwei Nukleinsäuren, die für unterschiedliche Fusionsproteine kodieren, kovalent miteinander verbunden sind.

12. Vektor enthaltend ein Proteingemisch nach einem der Ansprüche 1 bis 9 und/oder ein Nukleinsäuregemisch nach einem der Ansprüche 10 oder 11.

13. Zelle enthaltend ein Proteingemisch nach einem der Ansprüche 1 bis 9, ein Nukleinsäuregemisch nach einem der Ansprüche 10 oder 11 und/oder einen Vektor nach Anspruch 12.

14. Bibliothek enthaltend mindestens zwei Proteingemische nach einem der Ansprüche 1 bis 9, mindestens zwei Vektoren nach Anspruch 12 und/oder mindestens zwei Zellen nach Anspruch 13, wobei die Proteine oder Proteinfragmente der jeweiligen ersten oder jeweiligen zweiten Fusionsproteine voneinander unterschiedlich sind.

15. Verfahren zum Auffinden von Substanzen, die an ein Proteingemisch nach einem der Ansprüche 1 bis 9, an einen Vektor nach Anspruch 12 oder an eine Zelle nach Anspruch 13 binden, enthaltend die Schritte:
a) In-Kontaktbringen mindestens einer potentiell bindenden Substanz mit einem Proteingemisch nach einem der Ansprüche 1 bis 9, einem Vektor nach Anspruch 12 und/oder einer Zelle nach Anspruch 13 und
b) Messen der Bindung der potentiell bindenden Substanz an das Proteingemisch, den Vektor und/oder die Zelle.

16. Verfahren zum Auffinden von Proteinen oder Proteinfragmenten, die an eine Testsubstanz binden, enthaltend die Schritte:
a) In-Kontaktbringen mindestens einer Testsubstanz mit einer Bibliothek nach Anspruch 14 und
b) Messen der jeweiligen Bindung der Testsubstanz an die unterschiedlichen Proteingemische, Vektoren und/oder Zellen der Bibliothek.

17. Verfahren nach Anspruch 16 enthaltend die weiteren Schritte:
a) Auswählen mindestens eines Proteingemisches, eines Vektors oder einer Zelle auf Grund der gemessenen Bindung und
b) Herstellen einer zweiten Bibliothek, wobei die Bibliothek durch Modifikation des im ausgewählten Proteingemisch, im ausgewählten Vektor oder in der ausgewählten Zelle enthaltenden Proteins oder Proteinfragments erzeugt wird.

18. Verfahren nach Anspruch 16 enthaltend die weiteren Schritte:
a) Auswählen mindestens eines Proteingemisches, eines Vektors oder einer Zelle auf Grund der gemessenen Bindung,
b) Herstellen einer zweiten Bibliothek, wobei die Bibliothek durch Modifikation des im ausgewählten Proteingemisch, im ausgewählten Vektor oder in der ausgewählten Zelle enthaltenden Proteins oder Proteinfragments erzeugt wird,
c) In-Kontaktbringen mindestens einer Testsubstanz mit der zweiten Bibliothek,
d) Messen der jeweiligen Bindung der Testsubstanz an die unterschiedlichen Proteingemische, Vektoren oder Zellen der zweiten Bibliothek und
e) gegebenenfalls Wiederholen der Schritte a) bis d) bis ein Proteingemisch, ein Vektor oder eine Zelle ausgewählt wird, das, der bzw. die die gewünschte Bindung zeigt.

19. Verfahren nach einem der Ansprüche 15 bis 18, wobei in einem weiteren Schritt die bindende Substanz oder das in dem ausgewählten Proteingemisch, in dem ausgewählten Vektor oder in der ausgewählten Zelle enthaltende Protein oder Proteinfragment oder eine Variante davon mit einem pharmazeutisch akzeptablen Träger und/oder Hilfsstoff gemischt wird.

20. Kit zur Herstellung eines Nukleinsäuregemisches nach Anspruch 10 enthaltend:
a) mindestens eine erste Nukleinsäure enthaltend mindestens eine Restriktionsschnittstelle 5' und/oder 3' von einer Nukleinsäure kodierend für ein erstes Fusionsprotein enthaltend:
i) eine Interaktionsdomäne und
ü) eine Proteintranslokationssequenz, die bewirkt, dass das erste Fusionsprotein bei Expression in einem Bakterium in einem im wesentlichen gefalteten Zustand durch die zytoplasmatische Membran transloziert wird
b) mindestens eine zweite Nukleinsäure enthaltend mindestens eine Restriktionsschnittstelle 5' und/oder 3' von einer Nukleinsäure kodierend für ein zweites Fusionsprotein enthaltend:
i) eine Interaktionsdomäne und
ii) eine Proteintranslokationssequenz, die bewirkt, dass das zweite Fusionsprotein bei Expression in einem Bakterium in einem im wesentlichen ungefalteten Zustand durch die zytoplasmatische Membran transloziert wird,
wobei die Interaktionsdomäne des ersten Fusionsproteins an die des zweiten Fusionsproteins binden kann.

21. Verwendung einer Zelle nach Anspruch 13 zur Herstellung eines Proteingemisches nach einem der Ansprüche 1-9.

22. Verwendung eines Proteingemisches nach einem der Ansprüche 1 bis 9, eines Vektors nach Anspruch 12 und/oder einer Zelle nach Anspruch 13 zur Herstellung einer Bibliothek nach Anspruch 14.

## Claims

1. Protein mixture comprising:
a) at least a first fusion protein comprising:
i) a protein or protein fragment,
ii) an interaction domain and
iii) a protein translocation sequence which effects that the fusion protein upon expression in a bacterium is translocated through the cytoplasmic membrane in an essentially unfolded state,
and
b) at least a second fusion protein comprising:
i) a protein or protein fragment,
ii) an interaction domain and
iii) a protein translocation sequence which effects that the fusion protein upon expression in a bacterium is translocated through the cytoplasmic membrane in an essentially folded state,
wherein the interaction domain of the first fusion protein can bind to that of the second fusion protein.

2. Protein mixture according to claim 1, wherein the protein or protein fragment of the first fusion protein is an immunoglobulin heavy chain, an immunoglobulin light chain, a single-chain antibody, a diabody, a receptor, a receptor ligand, an integrin, an intimin, a carbohydrate- binding protein, an albumin-binding protein or protein A.

3. Protein mixture according to claim 1 or 2, wherein the protein or protein fragment of the second fusion protein is an autofluorescent protein, in particular GFP or a variant thereof, en enzyme, a cofactor-dependent protein, a protein that is encoded by a cDNA derived from a cDNA library or a synthetic protein.

4. Protein mixture according to claim 1, wherein the protein or the protein fragment of the first fusion protein and the protein translocation sequence is a phage coat protein, a periplasmatic marker enzyme, an intimin, a protein of the outer bacterial membrane or a periplasmatic receptor protein.

5. Protein mixture according to claim 4, wherein the phage coat protein is selected from the M13 phage coat proteins pIII, pVI, pVII, pVIII and pIX.

6. Protein mixture according to claims 1 to 5, wherein the interaction domains of the first and the second fusion protein are each respectively a leucine zipper domain and a leucine zipper domain, a helix-loop-helix-domain and a helix-loop-helix-domain, a calmodulin and a calmodulin binding peptide or a peptid dimer pair of naturally or synthetic origin.

7. Protein mixture according to one of claims 1 to 6, wherein the protein translocation sequence of the first fusion protein is a Sec-dependent, a SRP-dependent, a YidC-dependent sequence or a transport pathway-independent sequence which is integrated into the membrane.

8. Protein mixture according to one of claims 1 to 7, wherein the protein translocation sequence of the second fusion protein is a Tat-dependent or Thylakoid-Δ-pH-dependent sequence.

9. Protein mixture according to one of claims 1 to 8, wherein the first fusion-protein is covalently or non-covalently bound to the second fusion protein.

10. Nucleic acid mixture coding for a protein mixture according to one of claims 1 to 8.

11. Nucleic acid mixture according to claim 10, wherein at least two nucleic acids which code for different fusion proteins are covalently attached to each other.

12. Vector comprising a protein mixture according to one of claims 1 to 9 and/or a nucleic acid mixture according to one of claims 10 or 11.

13. Cell comprising a protein mixture according one of claims 1 to 9, a nucleic acid mixture according to one of claims 10 or 11 and/or a vector according to claim 12.

14. Library comprising at least two protein mixtures according to one of claims 1 to 9, at least two vectors according to claim 12 and/or at least two cells according to claim 13, wherein the proteins or protein fragments of the respective first or the respective second fusion proteins are different from each other.

15. Method of identifying a substance, which can bind to a protein mixture according to one of claims 1 to 9, to a vector according to claim 12 or to a cell according to claim 13 comprising the steps:
a) contacting at least one potentially binding substance with a protein mixture according to one of claims 1 to 9, a vector according to claim 12, and/or a cell according to claim 13 and
b) determining of the binding of the potentially binding substance to the protein mixture, the vector and/or the cell.

16. Method of identifying proteins or protein fragments, which bind to a test substance comprising the following steps:
a) contacting at least one test substance with a library according to claim 14 and
b) measuring of the respective binding of the test substance to the different protein mixtures, vectors and/or cells of the library.

17. A method according to claim 16 comprising the further steps:
a) selecting at least one protein mixture, one vector or one cell on the basis of the measured binding and
b) producing a second library wherein the library is generated by modification of the protein or protein fragment comprised in the selected protein mixture, in the selected vector or in the selected cell.

18. Method according to claim 16 comprising the further steps:
a) selecting at least one protein mixture, one vector or one cell on the basis of the measured binding,
b) producing a second library wherein the library is generated by modification of the proteins or protein fragment comprised in the selected protein mixture, in the selected vector or in the selected cell,
c) contacting at least one test substance with the second library,
d) measuring of the respective binding of the test substance to the different protein mixtures, vectors or cells of the second library and
e) if necessary, repeating of steps a) to d) until a protein mixture, a vector or a cell is selected which exhibits the desired binding.

19. Method according to one of the claims 15 to 18, wherein in a further step the binding substance or the protein or protein fragment or a variant thereof comprised in the selected protein mixture, in the selected vector or in the selected cell is mixed with a pharmaceutically acceptable carrier and/or excipient.

20. Kit for producing a nucleic acid mixture according to claim 10 comprising:
a) at least a first nucleic acid comprising at least a restriction site 5' and/or 3' of a nucleic acid coding for a first fusion protein comprising:
i) an interaction domain and
ii) a protein translocation sequence which effects that the first fusion protein upon expression in a bacterium is translocated through the cytoplasmic membrane in an essentially folded state
b) at least a second nucleic acid comprising at least a restriction site 5' and/or 3' of a nucleic acid coding for a second fusion protein comprising:
(i) an interaction domain and
(ii) a protein translocation sequence which effects that the second fusion protein upon expression in a bacterium is translocated through the cytoplasmic membrane in an essentially folded state.

21. Use of a cell according to claim 13 for the production of a protein mixture according to one of claims 1 to 9.

22. Use of a protein mixture according to one of claims 1 to 9, of a vector according to claim 12 and/or of a cell according to claim 13 for the production of the library according to claim 14.

## Revendications

1. Mélange de protéines contenant:
a) au moins une première protéine de fusion contenant:
i) une protéine ou un fragment de protéine,
ii) un domaine d'interaction et
iii) une séquence de translocation de protéine qui a pour effet que la protéine de fusion subit une translocation lors de l'expression dans une bactérie dans un état essentiellement non-enroulé à travers la membrane cytoplasmique,
et
b) au moins une deuxième protéine de fusion contenant:
i) une protéine ou un fragment de protéine,
ii) un domaine d'interaction et
iii) une séquence de translocation de protéine qui a pour effet que la protéine de fusion subit une translocation lors de l'expression dans une bactérie dans un état essentiellement enroulé à travers la membrane cytoplasmique,
tandis que le domaine d'interaction de la première protéine de fusion peut être lié à celui de la deuxième protéine de fusion.

2. Mélange de protéines selon la revendication 1, dans lequel la protéine ou le fragment de protéine de la première protéine de fusion est une chaîne lourde d'immunoglobuline, une chaîne légère d'immunoglobuline, un anticorps à chaîne unique, un "diabody", un récepteur, un ligand de récepteur, une intégrine, une intimine, une protéine de liaison aux hydrates de carbone, une protéine de liaison à l'albumine ou une protéine A.

3. Mélange de protéines selon la revendication 1 ou 2, dans lequel la protéine ou le fragment de protéine de la deuxième protéine de fusion est une protéine auto-fluorescente, en particulier une GFP ou une variante de celle-ci, une enzyme, une protéine dépendant d'un cofacteur, une protéine qui est codée par un ADNc provenant d'une bibliothèque d'ADNc, ou une protéine synthétique.

4. Mélange de protéines selon la revendication 1, dans lequel la protéine ou le fragment de protéine de la première protéine de fusion et la séquence de translocation de protéine est une protéine d'enveloppe de phage, une enzyme de marqueur périplasmatique, une intimine, une protéine de la membrane externe des bactéries ou une protéine de récepteur périplasmatique.

5. Mélange de protéines selon la revendication 4, dans lequel la protéine d'enveloppe de phage est choisie parmi les protéines d'enveloppe de phage-M13 pIII, pVI, pVII, pVIII et pIX.

6. Mélange de protéines selon l'une des revendications 1 à 5, dans lequel les domaines d'interaction de la première et de la deuxième protéines de fusion sont à chaque fois un domaine de fermeture éclair à leucine et un domaine de fermeture éclair à leucine, un domaine en hélice-boucle-hélice et un domaine en hélice-boucle-hélice, une calmoduline et un peptide de liaison à la calmoduline ou une paire de dimères peptidiques d'origine naturelle ou synthétique.

7. Mélange de protéines selon l'une des revendications 1 à 6, dans lequel la séquence de translocation de protéine de la première protéine de fusion est une séquence dépendante de Sec, dépendante de SRP, dépendante de YidC ou une séquence indépendante de la voie de transport, qui est intégrée dans la membrane.

8. Mélange de protéines selon l'une des revendications 1 à 7, dans lequel la séquence de translocation de protéine de la deuxième protéine de fusion est une séquence dépendante de Tat ou dépendante du ΔpH de thylakoïde.

9. Mélange de protéines selon l'une des revendications 1 à 8, dans lequel la première protéine de fusion est liée de manière covalente ou non-covalente à la deuxième protéine de fusion.

10. Mélange d'acides nucléiques codant pour un mélange de protéines selon l'une des revendications 1 à 8.

11. Mélange d'acides nucléiques selon la revendication 10, dans lequel au moins deux acides nucléiques qui codent pour des protéines de fusion différentes sont liés entre eux de manière covalente.

12. Vecteur contenant un mélange de protéines selon l'une des revendications 1 à 9 et/ou un mélange d'acides nucléiques selon l'une des revendications 10 ou 11.

13. Cellule contenant un mélange de protéines selon l'une des revendications 1 à 9, un mélange d'acides nucléiques selon l'une des revendications 10 ou 11 et/ ou un vecteur selon la revendication 12.

14. Bibliothèque contenant au moins deux mélanges de protéines selon l'une des revendications 1 à 9, au moins deux vecteurs selon la revendication 12 et/ ou au moins deux cellules selon la revendication 13, tandis que les protéines ou les fragments de protéines respectivement des premières ou des deuxièmes protéines de fusion sont différentes les unes des autres.

15. Procédé pour l'identification de substances qui se fixent à un mélange de protéines selon l'une des revendications 1 à 9, à un vecteur selon la revendication 12 ou à une cellule selon la revendication 13, contenant les étapes de:
a) mise en contact d'au moins une substance se fixant potentiellement avec un mélange de protéines selon l'une des revendications 1 à 9, un vecteur selon la revendication 12 et/ ou une cellule selon la revendication 13 et
b) mesure de la liaison de la substance se fixant potentiellement au mélange de protéines, au vecteur et/ ou à la cellule.

16. Procédé pour l'identification de protéines ou de fragments de protéines, qui se lient à une substance d'essai, comprenant les étapes de:
a) mise en contact d'au moins une substance d'essai avec une bibliothèque selon la revendication 14 et
b) mesure de la liaison respective de la substance d'essai aux mélanges de protéines différentes, aux vecteurs et/ ou aux cellules de la bibliothèque.

17. Procédé selon la revendication 16, comprenant les autres étapes:
a) sélection d'au moins un mélange de protéines, un vecteur ou une cellule sur la base de la liaison mesurée et
b) établissement d'une deuxième bibliothèque, tandis que la bibliothèque est élaborée par modification de la protéine ou du fragment de protéine contenu dans le mélange de protéines sélectionné, dans le vecteur sélectionné ou dans la cellule sélectionnée.

18. Procédé selon la revendication 16, comprenant les autres étapes:
a) sélection d'au moins un mélange de protéines, un vecteur ou une cellule sur la base de la liaison mesurée,
b) établissement d'une deuxième bibliothèque, tandis que la bibliothèque est élaborée par modification de la protéine ou du fragment de protéine contenu dans le mélange de protéines sélectionné, dans le vecteur sélectionné ou dans la cellule sélectionnée,
c) mise en contact d'au moins une substance d'essai avec la deuxième bibliothèque,
d) mesure de la liaison respectivement de la substance d'essai avec les mélanges de protéines différentes, les vecteurs ou les cellules de la deuxième bibliothèque et
e) éventuellement répétition des étapes a) à d) jusqu'à ce que soit sélectionné un mélange de protéines, un vecteur ou une cellule présentant la liaison souhaitée.

19. Procédé selon l'une des revendications 15 à 18, dans lequel dans une autre étape la substance se fixant ou la protéine ou le fragment de protéine contenu dans le mélange de protéines sélectionné, dans le vecteur sélectionné ou dans la cellule sélectionnée, ou leurs variantes, est mélangé avec un support et/ ou un adjuvant pharmaceutiquement acceptable.

20. Kit pour la préparation d'un mélange d'acides nucléiques selon la revendication 10 contenant:
a) au moins un premier acide nucléique contenant au moins une position de coupure de restriction 5' et/ou 3' d'un acide nucléique codant pour une première protéine de fusion contenant:
i) un domaine d'interaction et
ii) une séquence de translocation de protéine qui agit de telle sorte que la première protéine de fusion subit une translocation lors de l'expression dans une bactérie dans un état essentiellement enroulé à travers la membrane cytoplasmique
b) au moins un deuxième acide nucléique contenant au moins une position de coupure de restriction 5' et/ou 3' d'un acide nucléique codant pour une deuxième protéine de fusion, contenant:
i) un domaine d'interaction et
ii) une séquence de translocation de protéine qui agit de telle sorte que la première protéine de fusion subit une translocation lors de l'expression dans une bactérie dans un état essentiellement non-enroulé à travers la membrane cytoplasmique,
tandis que le domaine d'interaction de la première protéine de fusion peut être lié à celui de la deuxième protéine de fusion.

21. Utilisation d'une cellule selon la revendication 13 pour la préparation d'un mélange de protéines selon l'une des revendications 1-9.

22. Utilisation d'un mélange de protéines selon l'une des revendications 1 à 9, d'un vecteur selon la revendication 12 et/ ou d'une cellule selon la revendication 13 pour la préparation d'une bibliothèque selon la revendication 14.


### SEQUENZPROTOKOLL

<110> Universitätsklinikum Charité
<120> Gemisch mindestens zweier Fusionsproteine sowie ihre Herstellung und Verwendung .
<130> U30038
<150> DE 10256669.0
   <151> 2002-12-04
<160> 4
<170> Word 98, Windows
<210> 1
   <211> 4765
   <212> DNA
   <213> künstliche Sequenz
<220> .
   <221> pCD4/GFP24 Klonierungs- und Expressionsvektor
<400> 1
   ctagataaga aggaagaaaa ataatgaaca ataacgatct ctttcaggca tcacgtcggc   60
   gttttctggc acaactcggc ggcttaaccg tcgccgggat gctggggccg tcattgttaa  120
   cgccgcgacg tgcgactgcg gcccagccgg ccatggcggg atccgttcaa ctagcagacc  180
   attatcaaca aaatactcca attggcgatg gccctgtcct tttaccagac aaccattacc  240
   tgtcgacaca atctgccctt tcgaaagatc ccaacgaaaa gcgtgaccac atggtccttc  300
   ttgagtttgt aactgctgct gggatttccg gtggtggtgg tgctaccccg caggacctga  360
   acaccatgct gggtggtggt ggtagtaaag gagaagaact tttcactgga gttgtcccaa  420
   ttcttgttga attagatggt gatgttaatg ggcacaaatt ttctgtcagt ggagagggtg  480
   aaggtgatgc aacatacgga aaacttaccc ttaaatttat ttgcactact ggaaaactac  540
   ctgttccatg gccaacactt gtcactactt tctcttatgg tgttcaatgc ttttcccgtt  600
   atccggatca tatgaaacgg catgactttt tcaagagtgc catgcccgaa ggttatgtac  660
   aggaacgcac tatatctttc aaagatgacg ggaactacaa gacgcgtgct gaagtcaagt  720
   ttgaaggtga tacccttgtt aatcgtatcg agttaaaagg tattgatttt aaagaagatg  780
   gaaacattct cggacacaaa ctcgagtaca actataactc acacaatgta tacatcacgg  840
   cagacaaaca aaagaatgga atcaaagcta acttcaaaat tcgccacaac attgaagatt  900
   cggcctcggg ggccgcagaa caaaaactca tctcagaaga gaatctgtat ttccagggcg  960
   atgcttgcgg tggcaccgac accctgcaag ctgaaaccga ccagctggaa gacgagaaat 1020
   ccgctctgca gactgaaatc gctaacctgc tgaaagagaa agagaaactg gaattcattc 1080
   tggctgctca cggcggttgt gggctaggct aataacttaa gccaaggagg aaaataaaat 1140
   gaaataccta ttgcctacgg cagccgctgg attgttatta ctcgcggcac agccggccat 1200
   ggcaagcatc tgcggtggcc gtatcgctcg tctggaagaa aaagttaaaa ccctgaaagc 1260
   tcagaactcc gaactggctt ccaccgctaa catgctgcgt gaacaggttg ctcagctgaa 1320
   gcagaaagtt atgaaccacg gcggttgtgg tggcggttcc ctagcgggct ccggttccgg 1380
   tgattttgat tatgaaaaaa tggcaaacgc taataagggg gctatgaccg aaaatgccga 1440
   tgaaaacgcg ctacagtctg acgctaaagg caaacttgat tctgtcgcta ctgattacgg 1500
   tgctgctatc gatggtttca ttggtgacgt ttccggcctt gctaatggta atggtgctac 1560
   tggtgatttt gctggctcta attcccaaat ggctcaagtc ggtgacggtg ataattcacc 1620
   tttaatgaat aatttccgtc aatatttacc ttctttgcct cagtcggttg aatgtcgccc 1680
   ttatgtcttt ggcgctggta aaccatatga attttctatt gattgtgaca aaataaactt 1740
   attccgtggt gtctttgcgt ttcttttata tgttgccacc tttatgtatg tattttcgac 1800
   gtttgctaac atactgcgta ataaggagtc ttaataagct tgacctgtga agtgaaaaat 1860
   ggcgcacatt gtgcgacatt ttttttgtct gccgtttacc gctactgcgt cacggatctc 1920
   cacgcgccct gtagcggcgc attaagcgcg gcgggtgtgg tggttacgcg cagcgtgacc 1980
   gctacacttg ccagcgccct agcgcccgct cctttcgctt tcttcccttc ctttctcgcc 2040
   acgttcgccg gctttccccg tcaagctcta aatcgggggc tccctttagg gttccgattt 2100
   agtgctttac ggcacctcga ccccaaaaaa cttgattagg gtgatggttc acgtagtggg 2160
   ccatcgccct gatagacggt ttttcgccct ttgacgttgg agtccacgtt ctttaatagt 2220
   ggactcttgt tccaaactgg aacaacactc aaccctatct cggtctattc ttttgattta 2280
   taagggattt tgccgatttc ggcctattgg ttaaaaaatg agctgattta acaaaaattt 2340
   aacgcgcatg ctaacaaaat attaaaaaac gcccggcggc aaccgagcgt taatagtgaa 2400
   gttaccatca cggaaaaagg ttatgctgct tttaagaccc actttcacat ttaagttgtt 2460
   tttctaatcc gcatatgatc aattcaaggc cgaataagaa ggctggctct gcaccttggt 2520
   gatcaaataa ttcgatagct tgtcgtaata atggcggcat actatcagta gtaggtgttt 2580
   ccctttcttc tttagcgact tgatgctctt gatcttccaa tacgcaacct aaagtaaaat 2640
   gccccactgc gctgagtgca tataatgcat tctctagtga aaaaccttgt tggcataaaa 2700
   aggctaattg attttcgaga gtttcatact gtttttctgt aggccgtgta cctaaatgta 2760
   cttttgctcc atcgcgatga cttagtaaag cacatctaaa acttttagcg ttattacgta 2820
   aaaaatcttg ccagctttcc ccttctaaag ggcaaaagtg agtatggtgc ctatctaaca 2880
   tctcaatggc taaggcgtcg agcaaagccc gcttattttt tacatgccaa tacaatgtag 2940
   gctgctctac acctagcttc tgggcgagtt tacgggttgt taaaccttcg attccgacct 3000
   cattaagcag ctctaatgcg ctgttaatca ctttactttt atctaaacga gacatcatta 3060
   attcctatta cgccccgccc tgccactcat cgcagtactg ttgtaattca ttaagcattc 3120
   tgccgacatg gaagccatca caaacggcat gatgaacctg aatcgccagc ggcatcagca 3180
   ccttgtcgcc ttgcgtataa tatttgccca tagtgaaaac gggggcgaag aagttgtcca 3240
   tattggccac gtttaaatca aaactggtga aactcaccca gggattggct gagacgaaaa 3300
   acatattctc aataaaccct ttagggaaat aggccaggtt ttcaccgtaa cacgccacat 3360
   cttgcgaata tatgtgtaga aactgccgga aatcgtcgtg gtattcactc cagagcgatg 3420
   aaaacgtttc agtttgctca tggaaaacgg tgtaacaagg gtgaacacta tcccatatca 3480
   ccagctcacc gtctttcatt gccatacgga attccggatg agcattcatc aggcgggcaa 3540
   gaatgtgaat aaaggccgga taaaacttgt gcttattttt ctttacggtc tttaaaaagg 3600
   ccgtaatatc cagctgaacg gtctggttat aggtacattg agcaactgac tgaaatgcct 3660
   caaaatgttc tttacgatgc cattgggata tatcaacggt ggtatatcca gtgatttttt 3720
   tctccatact cttccttttt caatattatt gaagcattta tcagggttat tgtctcatga 3780
   gcggatacat atttgaatgt atttagaaaa ataaacaaat aggggttccg cgcacatttc 3840
   cccgaaaagt gccacctgaa attgtaagcg ttactagttt aaaaggatct aggtgaagat 3900
   cctttttgat aatctcatga ccaaaatccc ttaacgtgag ttttcgttcc actgagcgtc 3960
   agaccccgta gaaaagatca aaggatcttc ttgagatcct ttttttctgc gcgtaatctg 4020
   ctgcttgcaa acaaaaaaac caccgctacc agcggtggtt tgtttgccgg atcaagagct 4080
   accaactctt tttccgaagg taactggctt cagcagagcg cagataccaa atactgtcct 4140
   tctagtgtag ccgtagttag gccaccactt caagaactct gtagcaccgc ctacatacct 4200
   cgctctgcta atcctgttac cagtggctgc tgccagtggc gataagtcgt gtcttaccgg 4260
   gttggactca agacgatagt taccggataa ggcgcagcgg tcgggctgaa cggggggttc 4320
   gtgcacacag cccagcttgg agcgaacgac ctacaccgaa ctgagatacc tacagcgtga 4380
   gctatgagaa agcgccacgc ttcccgaagg gagaaaggcg gacaggtatc cggtaagcgg 4440
   cagggtcgga acaggagagc gcacgaggga gcttccaggg ggaaacgcct ggtatcttta 4500
   tagtcctgtc gggtttcgcc acctctgact tgagcgtcga tttttgtgat gctcgtcagg 4560
   ggggcggagc ctatggaaaa acgccagcaa cgcggccttt ttacggttcc tggccttttg 4620
   ctggcctttt gctcacatga cccgacacca tcgaatggcc agatgattaa ttcctaattt 4680
   ttgttgacac tctatcattg atagagttat tttaccactc cctatcagtg atagagaaaa 4740
   gtgaaatgaa tagttcgaca aaaat                                       4765
<210> 2
   <211> 4971
   <212> DNA
   <213> künstliche Sequenz
<220>
   <221> pCAl/GFP24 Klonierungs- und Expressionsvektor
<400> 2
   ctagataaga aggaagaaaa ataatgaaca ataacgatct ctttcaggca tcacgtcggc    60
   gttttctggc acaactcggc ggcttaaccg tcgccgggat gctggggccg tcattgttaa   120
   cgccgcgacg tgcgactgcg gcccagccgg ccatggcggg atccgttcaa ctagcagacc   180
   attatcaaca aaatactcca attggcgatg gccctgtcct tttaccagac aaccattacc   240
   tgtcgacaca atctgccctt tcgaaagatc ccaacgaaaa gcgtgaccac atggtccttc   300
   ttgagtttgt aactgctgct gggatttccg gtggtggtgg tgctaccccg caggacctga   360
   acaccatgct gggtggtggt ggtagtaaag gagaagaact tttcactgga gttgtcccaa   420
   ttcttgttga attagatggt gatgttaatg ggcacaaatt ttctgtcagt ggagagggtg   480
   aaggtgatgc aacatacgga aaacttaccc ttaaatttat ttgcactact ggaaaactac   540
   ctgttccatg gccaacactt gtcactactt tctcttatgg tgttcaatgc ttttcccgtt    600
   atccggatca tatgaaacgg catgactttt tcaagagtgc catgcccgaa ggttatgtac    660
   aggaacgcac tatatctttc aaagatgacg ggaactacaa gacgcgtgct gaagtcaagt    720
   ttgaaggtga tacccttgtt aatcgtatcg agttaaaagg tattgatttt aaagaagatg    780
   gaaacattct cggacacaaa ctcgagtaca actataactc acacaatgta tacatcacgg    840
   cagacaaaca aaagaatgga atcaaagcta acttcaaaat tcgccacaac attgaagatt    900
   cggcctcggg ggccgcagaa caaaaactca tctcagaaga gaatctgtat ttccagggcg    960
   ggcccaaacc ttccaccccg cctggttctt caggcgcctg cggtggcctg accgacaccc   1020
   tgcaagctga aaccgaccag ctggaagacg agaaatccgc tctgcagact gaaatcgcta   1080
   acctgctgaa agagaaagag aaactggaat tcattctggc tgctcacggc ggttgttaat   1140
   aacttaagcc aaggaggaaa ataaaatgaa atacctattg cctacggcag ccgctggatt   1200
   gttattactc gctgcccaac cagcgatggc cgcacaggtt aaactgctcg agagcgcttg   1260
   cggtggccgt atcgctcgtc tggaagaaaa agttaaaacc ctgaaagctc agaactccga   1320
   actggcttcc accgctaaca tgctgcgtga acaggttgct cagctgaagc agaaagttat   1380
   gaaccacggc ggttgtgcta gcggtggcgg ctccggttcc ggtgattttg attatgaaaa   1440
   aatggcaaac gctaataagg gggctatgac cgaaaatgcc gatgaaaacg cgctacagtc   1500
   tgacgctaaa ggcaaacttg attctgtcgc tactgattac ggtgctgcta tcgatggttt   1560
   cattggtgac gtttccggcc ttgctaatgg taatggtgct actggtgatt ttgctggctc   1620
   taattcccaa atggctcaag tcggtgacgg tgataattca cctttaatga ataatttccg   1680
   tcaatattta ccttctttgc ctcagtcggt tgaatgtcgc ccttatgtct ttggcgctgg   1740
   taaaccatat gaattttcta ttgattgtga caaaataaac ttattccgtg gtgtctttgc   1800
   gtttctttta tatgttgcca cctttatgta tgtattttcg acgtttgcta acatactgcg   1860
   taataaggag tcttaataag cttgacctgt gaagtgaaaa atggcgcaca ttgtgcgaca   1920
   ttttttttgt ctgccgttta ccgctactgc gtcacggatc tccacgcgcc ctgtagcggc   1980
   gcattaagcg cggcgggtgt ggtggttacg cgcagcgtga ccgctacact tgccagcgcc   2040
   ctagcgcccg ctcctttcgc tttcttccct tcctttctcg ccacgttcgc cggctttccc   2100
   cgtcaagctc taaatcgggg gctcccttta gggttccgat ttagtgcttt acggcacctc   2160
   gaccccaaaa aacttgatta gggtgatggt tcacgtagtg ggccatcgcc ctgatagacg   2220
   gtttttcgcc ctttgacgtt ggagtccacg ttctttaata gtggactctt gttccaaact   2280
   ggaacaacac tcaaccctat ctcggtctat tcttttgatt tataagggat tttgccgatt   2340
   tcggcctatt ggttaaaaaa tgagctgatt taacaaaaat ttaacgcgaa ttttaacaaa   2400
   atattaacgc ttacaatttc aggtggcact tttcggggaa atgtgcgcgg aacccctatt   2460
   tgtttatttt tctaaataca ttcaaatatg tatccgctca tgagacaata accctgataa   2520
   atgcttcaat aatattgaaa aaggaagagt atgagtattc aacatttccg tgtcgccctt   2580
   attccctttt ttgcggcatt ttgccttcct gtttttgctc acccagaaac gctggtgaaa   2640
   gtaaaagatg ctgaagatca gttgggtgca cgagtgggtt acatcgaact ggatctcaac   2700
   agcggtaaga tccttgagag ttttcgcccc gaagaacgtt ttccaatgat gagcactttt   2760
   aaagttctgc tatgtggcgc ggtattatcc cgtattgacg ccgggcaaga gcaactcggt   2820
   cgccgcatac actattctca gaatgacttg gttgagtact caccagtcac agaaaagcat   2880
   cttacggatg gcatgacagt aagagaatta tgcagtgctg ccataaccat gagtgataac   2940
   actgcggcca acttacttct gacaacgatc ggaggaccga aggagctaac cgcttttttg   3000
   cacaacatgg gggatcatgt aactcgcctt gatcgttggg aaccggagct gaatgaagcc   3060
   ataccaaacg acgagcgtga caccacgatg cctgtagcaa tggcaacaac gttgcgcaaa   3120
   ctattaactg gcgaactact tactctagct tcccggcaac aattgataga ctggatggag   3180
   gcggataaag ttgcaggacc acttctgcgc tcggcccttc cggctggctg gtttattgct   3240
   gataaatctg gagccggtga gcgtggctct cgcggtatca ttgcagcact ggggccagat   3300
   ggtaagccct cccgtatcgt agttatctac acgacgggga gtcaggcaac tatggatgaa   3360
   cgaaatagac agatcgctga gataggtgcc tcactgatta agcattggta ggaattaatg   3420
   atgtctcgtt tagataaaag taaagtgatt aacagcgcat tagagctgct taatgaggtc   3480
   ggaatcgaag gtttaacaac ccgtaaactc gcccagaagc taggtgtaga gcagcctaca   3540
   ttgtattggc atgtaaaaaa taagcgggct ttgctcgacg ccttagccat tgagatgtta   3600
   gataggcacc atactcactt ttgcccttta gaaggggaaa gctggcaaga ttttttacgt    3660
   aataacgcta aaagttttag atgtgcttta ctaagtcatc gcgatggagc aaaagtacat   3720
   ttaggtacac ggcctacaga aaaacagtat gaaactctcg aaaatcaatt agccttttta    3780
   tgccaacaag gtttttcact agagaatgca ttatatgcac tcagcgcagt ggggcatttt   3840
   actttaggtt gcgtattgga agatcaagag catcaagtcg ctaaagaaga aagggaaaca   3900
   cctactactg atagtatgcc gccattatta cgacaagcta tcgaattatt tgatcaccaa   3960
   ggtgcagagc cagccttctt attcggcctt gaattgatca tatgcggatt agaaaaacaa   4020
   cttaaatgtg aaagtgggtc ttaaaagcag cataaccttt ttccgtgatg gtaacttcac   4080
   tagtttaaaa ggatctaggt gaagatcctt tttgataatc tcatgaccaa aatcccttaa   4140
   cgtgagtttt cgttccactg agcgtcagac cccgtagaaa agatcaaagg atcttcttga   4200
   gatccttttt ttctgcgcgt aatctgctgc ttgcaaacaa aaaaaccacc gctaccagcg   4260
   gtggtttgtt tgccggatca agagctacca actctttttc cgaaggtaac tggcttcagc   4320
   agagcgcaga taccaaatac tgtccttcta gtgtagccgt agttaggcca ccacttcaag  4380
   aactctgtag caccgcctac atacctcgct ctgctaatcc tgttaccagt ggctgctgcc  4440
   agtggcgata agtcgtgtct taccgggttg gactcaagac gatagttacc ggataaggcg  4500
   cagcggtcgg gctgaacggg gggttcgtgc acacagccca gcttggagcg aacgacctac  4560
   accgaactga gatacctaca gcgtgagcta tgagaaagcg ccacgcttcc cgaagggaga  4620
   aaggcggaca ggtatccggt aagcggcagg gtcggaacag gagagcgcac gagggagctt  4680
   ccagggggaa acgcctggta tctttatagt cctgtcgggt ttcgccacct ctgacttgag  4740
   cgtcgatttt tgtgatgctc gtcagggggg cggagcctat ggaaaaacgc cagcaacgcg  4800
   gcctttttac ggttcctggc cttttgctgg ccttttgctc acatgacccg acaccatcga  4860
   atggccagat gattaattcc taatttttgt tgacactcta tcattgatag agttatttta  4920
   ccactcccta tcagtgatag agaaaagtga aatgaatagt tcgacaaaaa t           4971
<210> 3
   <211> 4765
   <212> DNA
   <213> künstliche Sequenz
<220>
   <221> pCN1/GFP24 Klonierungs- und Expressionsvektor
<400> 3
   ctagataaga aggaagaaaa ataatgaaca ataacgatct ctttcaggca tcacgtcggc   60
   gttttctggc acaactcggc ggcttaaccg tcgccgggat gctggggccg tcattgttaa  120
   cgccgcgacg tgcgactgcg gcccagccgg ccatggcggg atccgttcaa ctagcagacc  180
   attatcaaca aaatactcca attggcgatg gccctgtcct tttaccagac aaccattacc  240
   tgtcgacaca atctgccctt tcgaaagatc ccaacgaaaa gcgtgaccac atggtccttc  300
   ttgagtttgt aactgctgct gggatttccg gtggtggtgg tgctaccccg caggacctga  360
   acaccatgct gggtggtggt ggtagtaaag gagaagaact tttcactgga gttgtcccaa  420
   ttcttgttga attagatggt gatgttaatg ggcacaaatt ttctgtcagt ggagagggtg  480
   aaggtgatgc aacatacgga aaacttaccc ttaaatttat ttgcactact ggaaaactac  540
   ctgttccatg gccaacactt gtcactactt tctcttatgg tgttcaatgc ttttcccgtt  600
   atccggatca tatgaaacgg catgactttt tcaagagtgc catgcccgaa ggttatgtac  660
   aggaacgcac tatatctttc aaagatgacg ggaactacaa gacgcgtgct gaagtcaagt  720
   ttgaaggtga tacccttgtt aatcgtatcg agttaaaagg tattgatttt aaagaagatg  780
   gaaacattct cggacacaaa ctcgagtaca actataactc acacaatgta tacatcacgg  840
   cagacaaaca aaagaatgga atcaaagcta acttcaaaat tcgccacaac attgaagatt  900
   cggcctcggg ggccgcagaa caaaaactca tctcagaaga gaatctgtat ttccagggcg  960
   atgcttgcgg tggcaccgac accctgcaag ctgaaaccga ccagctggaa gacgagaaat 1020
   ccgctctgca gactgaaatc gctaacctgc tgaaagagaa agagaaactg gaattcattc 1080
   tggctgctca cggcggttgt gggctaggct aataacttaa gccaaggagg aaaataaaat 1140
   gaaataccta ttgcctacgg cagccgctgg attgttatta ctcgcggcac agccggccat 1200
   ggcaagcatc tgcggtggcc gtatcgctcg tctggaagaa aaagttaaaa ccctgaaagc 1260
   tcagaactcc gaactggctt ccaccgctaa catgctgcgt gaacaggttg ctcagctgaa 1320
   gcagaaagtt atgaaccacg gcggttgtgg tggcggttcc ctagcgggct ccggttccgg 1380
   tgattttgat tatgaaaaaa tggcaaacgc taataagggg gctatgaccg aaaatgccga 1440
   tgaaaacgcg ctacagtctg acgctaaagg caaacttgat tctgtcgcta ctgattacgg 1500
   tgctgctatc gatggtttca ttggtgacgt ttccggcctt gctaatggta atggtgctac 1560
   tggtgatttt gctggctcta attcccaaat ggctcaagtc ggtgacggtg ataattcacc 1620
   tttaatgaat aatttccgtc aatatttacc ttctttgcct cagtcggttg aatgtcgccc 1680
   ttatgtcttt ggcgctggta aaccatatga attttctatt gattgtgaca aaataaactt 1740
   attccgtggt gtctttgcgt ttcttttata tgttgccacc tttatgtatg tattttcgac 1800
   gtttgctaac atactgcgta ataaggagtc ttaataagct tgacctgtga agtgaaaaat 1860
   ggcgcacatt gtgcgacatt ttttttgtct gccgtttacc gctactgcgt cacggatctc 1920
   cacgcgccct gtagcggcgc attaagcgcg gcgggtgtgg tggttacgcg cagcgtgacc 1980
   gctacacttg ccagcgccct agcgcccgct cctttcgctt tcttcccttc ctttctcgcc 2040
   acgttcgccg gctttccccg tcaagctcta aatcgggggc tccctttagg gttccgattt 2100
   agtgctttac ggcacctcga ccccaaaaaa cttgattagg gtgatggttc acgtagtggg 2160
   ccatcgccct gatagacggt ttttcgccct ttgacgttgg agtccacgtt ctttaatagt 2220
   ggactcttgt tccaaactgg aacaacactc aaccctatct cggtctattc ttttgattta 2280
   taagggattt tgccgatttc ggcctattgg ttaaaaaatg agctgattta acaaaaattt 2340
   aacgcgcatg caacgcttac aatttcaggt ggcacttttc ggggaaatgt gcgcggaacc 2400
   cctatttgtt tatttttcta aatacattca aatatgtatc cgctcatgag acaataaccc 2460
   tgataaatgc ttcaataata ttgaaaaagg aagagtatgg agaaaaaaat cactggatat 2520
   accaccgttg atatatccca atggcatcgt aaagaacatt ttgaggcatt tcagtcagtt 2580
   gctcaatgta cctataacca gaccgttcag ctggatatta cggccttttt aaagaccgta 2640
   aagaaaaata agcacaagtt ttatccggcc tttattcaca ttcttgcccg cctgatgaat 2700
   gctcatccgg aattccgtat ggcaatgaaa gacggtgagc tggtgatatg ggatagtgtt 2760
   cacccttgtt acaccgtttt ccatgagcaa actgaaacgt tttcatcgct ctggagtgaa 2820
   taccacgacg atttccggca gtttctacac atatattcgc aagatgtggc gtgttacggt 2880
   gaaaacctgg cctatttccc taaagggttt attgagaata tgtttttcgt ctcagccaat 2940
   ccctgggtga gtttcaccag ttttgattta aacgtggcca atatggacaa cttcttcgcc 3000
   cccgttttca ctatgggcaa atattatacg caaggcgaca aggtgctgat gccgctggcg 3060
   attcaggttc atcatgccgt ttgtgatggc ttccatgtcg gcagaatgct taatgaatta 3120
   caacagtact gcgatgagtg gcagggcggg gcgtaatagg aattaatgat gtctcgttta 3180
   gataaaagta aagtgattaa cagcgcatta gagctgctta atgaggtcgg aatcgaaggt 3240
   ttaacaaccc gtaaactcgc ccagaagcta ggtgtagagc agcctacatt gtattggcat 3300
   gtaaaaaata agcgggcttt gctcgacgcc ttagccattg agatgttaga taggcaccat 3360
   actcactttt gccctttaga aggggaaagc tggcaagatt ttttacgtaa taacgctaaa 3420
   agttttagat gtgctttact aagtcatcgc gatggagcaa aagtacattt aggtacacgg 3480
   cctacagaaa aacagtatga aactctcgaa aatcaattag cctttttatg ccaacaaggt 3540
   ttttcactag agaatgcatt atatgcactc agcgcagtgg ggcattttac tttaggttgc 3600
   gtattggaag atcaagagca tcaagtcgct aaagaagaaa gggaaacacc tactactgat 3660
   agtatgccgc cattattacg acaagctatc gaattatttg atcaccaagg tgcagagcca 3720
   gccttcttat tcggccttga attgatcata tgcggattag aaaaacaact taaatgtgaa 3780
   agtgggtctt aaaagcagca taaccttttt ccgtgatggt aacttcacta ttaacgctcg 3840
   gttgccgccg ggcgtttttt aatattttgt taactagttt aaaaggatct aggtgaagat 3900
   cctttttgat aatctcatga ccaaaatccc ttaacgtgag ttttcgttcc actgagcgtc 3960
   agaccccgta gaaaagatca aaggatcttc ttgagatcct ttttttctgc gcgtaatctg 4020
   ctgcttgcaa acaaaaaaac caccgctacc agcggtggtt tgtttgccgg atcaagagct 4080
   accaactctt tttccgaagg taactggctt cagcagagcg cagataccaa atactgtcct 4140
   tctagtgtag ccgtagttag gccaccactt caagaactct gtagcaccgc ctacatacct 4200
   cgctctgcta atcctgttac cagtggctgc tgccagtggc gataagtcgt gtcttaccgg 4260
   gttggactca agacgatagt taccggataa ggcgcagcgg tcgggctgaa cggggggttc 4320
   gtgcacacag cccagcttgg agcgaacgac ctacaccgaa ctgagatacc tacagcgtga 4380
   gctatgagaa agcgccacgc ttcccgaagg gagaaaggcg gacaggtatc cggtaagcgg 4440
   cagggtcgga acaggagagc gcacgaggga gcttccaggg ggaaacgcct ggtatcttta 4500
   tagtcctgtc gggtttcgcc acctctgact tgagcgtcga tttttgtgat gctcgtcagg 4560
   ggggcggagc ctatggaaaa acgccagcaa cgcggccttt ttacggttcc tggccttttg 4620
   ctggcctttt gctcacatga cccgacacca tcgaatggcc agatgattaa ttcctaattt 4680
   ttgttgacac tctatcattg atagagttat tttaccactc cctatcagtg atagagaaaa 4740
   gtgaaatgaa tagttcgaca aaaat                                        4765
<210> 4
   <211> 823
   <212> DNA
   <213> künstliche Sequenz
<220>
   <221> reife TEM-1 β-Lactamase Klonierungskassette
<400> 4